(19) Europäisches Patentamt
European Patent Office
Office européen des brevets

(11) Publication number: 0 322 133 B1

# (12) EUROPEAN PATENT SPECIFICATION

(45) Date of publication of patent specification: 22.05.91 Bulletin 91/21

(51) Int. Cl.[5]: C07D 239/95, A61K 31/505, C07D 401/04

(21) Application number: 88311461.3

(22) Date of filing: 02.12.88

## (54) Quinazoline derivatives.

(30) Priority: 03.12.87 GB 8728336
25.08.88 GB 8820184

(43) Date of publication of application: 28.06.89 Bulletin 89/26

(45) Publication of the grant of the patent: 22.05.91 Bulletin 91/21

(84) Designated Contracting States: AT BE CH DE ES FR GB GR IT LI LU NL SE

(56) References cited:
EP-A- 0 028 473
CH-A- 457 460
DE-C- 958 197
FR-A- 1 310 457
GB-A- 806 772
GB-A- 1 390 014
US-A- 3 635 979
US-A- 3 956 495
US-A- 4 098 788

(73) Proprietor: SmithKline Beecham Intercredit B.V.
28-34 Blaak P.O. Box 2
NL-3000 DG Rotterdam (NL)

(72) Inventor: Ife, Robert John
9 Edmonds Drive Aston Brook
Stevenage Hertfordshire (GB)
Inventor: Brown, Thomas Henry
17 Godfries Close
Tewin Hertfordshire (GB)
Inventor: Leach, Colin Andrew
30 Wellington Road
Stevenage Hertfordshire (GB)

(74) Representative: Giddings, Peter John, Dr. et al
Smith Kline & French Laboratories Ltd.
Corporate Patents Mundells
Welwyn Garden City Hertfordshire AL7 1EY (GB)

EP 0 322 133 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue Saint-Denis, 75001 PARIS

## Description

The present invention relates to substituted quinazoline derivatives, processes for their preparation, intermediates useful in their preparation, pharmaceutical compositions containing them and their use in therapy.

Substituted aminoquinazolines are known in the art. For example, GB 806722 and FR 1310457 disclose certain 2,4-diaminoquinazolines as antibacterial agents. In addition, US 3,635,979 and EP 028473-A1 disclose certain 2,4-diamino-6,7 or 6/7 alkoxy substituted quinazolines which are said to have activity as antihypertensive agents. However, none of the foregoing indicate that compounds of this structural class exhibit gastric acid secretion-inhibitory activity.

Accordingly the present invention provides, in a first aspect compounds of structure (I)

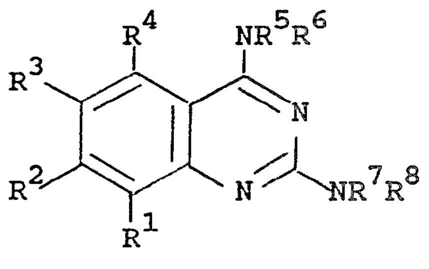

(I)

in which

$R^1$ to $R^4$ are the same or different and are each hydrogen, $C_{1-4}$alkyl, $C_{1-4}$alkoxy, phenyl, $C_{1-4}$alkylthio, $C_{1-4}$alkanoyl, amino, $C_{1-6}$alkylamino, di$C_{1-4}$alkylamino, halogen or trifluoromethyl provided that at least two of $R^1$ to $R^4$ are hydrogen.

$R^5$ and $R^6$ are the same, or different and are each hydrogen, $C_{1-4}$alkyl, $-(CH_2)_nAr$ in which n is 0 to 4 and Ar is a phenyl group optionally substituted by 1 to 3 substituents selected from $C_{1-4}$alkyl, $C_{1-4}$alkoxy, $C_{1-4}$alkylthio, halogen, cyano, amino, hydroxy, carbamoyl, carboxy, $C_{1-4}$alkanoyl or trifluoromethyl, or $R^5$ and $R^6$ together with the nitrogen atom to which they are attached form a saturated or unsaturated carbocyclic ring ; and

$R^7$ and $R^8$ are the same or different and are each hydrogen, $C_{1-4}$alkyl, $(CH_2)_nAr^1$ in which n is 0 to 4 and $Ar^1$ is a phenyl group optionally substituted by 1 to 3 substituents as described for Ar above, or $R^7$ and $R^8$ together with the nitrogen atom to which they are attached form a saturated or unsaturated carbocyclic ring ; and pharmaceutically acceptable salts thereof, provided that :

° $R^5$, $R^6$, $R^7$ and $R^8$ are not all, at the same time, hydrogen ;

° at least one of $R^5$, $R^6$, $R^7$ and $R^8$ is a $(CH_2)_nAr$ or $(CH_2)_nAr^1$ group as appropriate ; and

° when $R^1$ and $R^4$ are both hydrogen, and one of $R^2$ and $R^3$ is $C_{1-4}$alkoxy, the other is not hydrogen or $C_{1-4}$alkoxy.

Suitably at least two of $R^1$ to $R^4$ are hydrogen and the others are the same or different and are each hydrogen, $C_{1-4}$alkyl, $C_{1-4}$alkoxy, phenyl, $C_{1-4}$alkylthio, $C_{1-4}$alkanoyl, amino, $C_{1-4}$ alkylamino, di$C_{1-4}$alkylamino, halogen, trifluoromethyl or nitro. More suitably, three of $R^1$ to $R^4$ are hydrogen and the other is hydrogen, $C_{1-4}$ alkyl, $C_{1-4}$alkoxy, phenyl, $C_{1-4}$alkylthio, $C_{1-4}$alkanoyl, amino, $C_{1-4}$alkylamino, di$C_{1-4}$alkylamino, halogen or trifluoromethyl ; preferably $R^2$ to $R^4$ are hydrogen and $R^1$ is hydrogen, $C_{1-4}$alkyl, $C_{1-4}$alkoxy, phenyl, $C_{1-4}$alkylthio, $C_{1-4}$-alkanoyl, amino, $C_{1-4}$alkylamino, di$C_{1-4}$alkylamino, halogen or trifluoromethyl ; more preferably $R^2$ to $R^4$ are hydrogen and $R^1$ is hydrogen, $C_{1-4}$alkyl or $C_{1-4}$alkoxy ; most preferably $R^2$ to $R^4$ are hydrogen and $R^1$ is hydrogen or $C_{1-4}$alkoxy, in particular methoxy.

Suitably $R^5$ and $R^6$ are the same or different and are each hydrogen or $(CH_2)_nAr$ in which n is 0 to 4 and Ar is an optionally substituted phenyl group or $R^5$ and $R^6$ together with the nitrogen atom to which they are attached form a saturated or unsaturated carbocyclic ring. More suitably, one of $R^5$ and $R^6$ is hydrogen or $C_{1-4}$ alkyl and the other is hydrogen, $C_{1-4}$alkyl or $(CH_2)_nAr$. Most suitably one of $R^5$ and $R^6$ is hydrogen or $C_{1-4}$alkyl and the other is $(CH_2)_nAr$. Preferably one of $R^5$ and $R^6$ is $C_{1-4}$alkyl and the other is $(CH_2)_nAr$ ; most preferably one of $R^5$ and $R^6$ is $C_{1-4}$alkyl, in particular methyl and the other is $(CH_2)_n$ Ar in which n is 0.

Suitably, Ar is unsubstituted or substituted by 1 to 3 substituents selected from $C_{1-4}$alkyl, $C_{1-4}$alkoxy, $C_{1-4}$ alkylthio, halogen, cyano, amino, hydroxy, carbamoyl, carboxy, $C_{1-4}$ alkanoyl or trifluoromethyl. More suitably. Ar is unsubstituted or substituted by two substituents selected from hydrogen, $C_{1-4}$alkyl, $C_{1-4}$alkoxy, $C_{1-4}$alkylthio, halogen, cyano, amino, hydroxy, carbamoyl, carboxy, $C_{1-4}$alkanoyl or trifluoromethyl. More preferably, Ar is unsubstituted or substituted by two substituents selected from $C_{1-4}$alkyl and $C_{1-4}$alkoxy. Most preferably, Ar is unsubstituted or substituted by a single substituent selected from the above-noted groups, in particular $C_{1-4}$ alkyl or $C_{1-4}$alkoxy.

Suitably, $R^7$ and $R^8$ are the same or different and are each hydrogen, $C_{1-4}$alkyl, $(CH_2)_nAr^1$ in which n is 0

to 4 and $Ar^1$ is an optionally substituted phenyl group, or $R^7$ and $R^8$ together with the nitrogen atom to which they are attached form a saturated or unsaturated carbocyclic ring. More suitably one of $R^7$ and $R^8$ is hydrogen or $C_{1-4}$alkyl and the other is hydrogen, $C_{1-4}$alkyl or $(CH_2)_nAr^1$, or $R^7$ and $R^8$ together with the nitrogen atom to which they are attached form a saturated or unsaturated carbocyclic ring. Most suitably, one of $R^7$ and $R^8$ is hydrogen or $C_{1-4}$alkyl and the other is hydrogen, $C_{1-4}$alkyl or $(CH_2)_nAr^1$. Preferably one of $R^7$ and $R^8$ is hydrogen or $C_{1-4}$alkyl and the other is $(CH_2)_nAr^1$ ; more preferably one of $R^7$ and $R^8$ is hydrogen and the other is $(CH_2)_nAr^1$; most preferably, one of $R^7$ and $R^8$ is hydrogen and the other is $(CH_2)_nAr^1$ in which n is 0.

Suitably, the group $Ar^1$ is unsubstituted or optionally substituted by 1 to 3 substituents as hereinabove described for the group Ar in $R^5$. Preferably the group $Ar^1$ is unsubstituted or optionally substituted by two substituents as hereinabove described for Ar. Preferably the group $Ar^1$ is unsubstituted or substituted by two groups, which may be the same or different ; more preferably the group $Ar^1$ is unsubstituted or substituted by two groups which may be the same or different and selected from $C_{1-4}$alkyl and $C_{1-4}$alkoxy. Most preferably the group $Ar^1$ is unsubstituted or substituted by one or two groups, for example a $C_{1-4}$alkyl group, in particular a methyl group or a halogen atom, in particular a fluorine atom ; or a $C_{1-4}$alkyl group and a halogen atom in particular a methyl group and fluorine atom. Particularly preferably the group $Ar^1$ is substituted by a methyl group in the 2-position of the ring and a fluorine atom in the 4-position of the ring.

$C_{1-4}$alkyl groups (either alone or as part of another group) can be straight or branched.

It will be appreciated that compounds of structure (I) in which one or more of $R^1$ to $R^8$ is a $C_{3-4}$alkyl group (either alone or as part of another group) may contain an assymetric centre due to the presence of the $C_{3-4}$alkyl group. Such compounds will exist as two (or more) optical isomers (enantiomers). Both the pure enantiomers, racemic mixtures (50% of each enantiomer) and unequal mixtures of the two are included within the scope of the present invention. Further, all diastereomeric forms possible (pure enantiomers and mixtures thereof) are within the scope of the invention.

The compounds of the present invention can be prepared by processes analogous to those known in the art. The present invention therefore provides in a further aspect a process for the preparation of a compound of structure (I) or a pharmaceutically acceptable salt thereof which comprises

(a) reaction of a compound of structure (II)

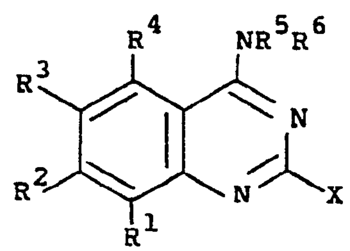

(II)

in which $R^1$ to $R^6$ are as described for structure (I) except that where necessary they are in protected form, and X is a group displaceable by an amine, with an amine of structure $R^7R^8NH$ in which $R^7$ and $R^8$ are as described for structure (I) ; or

(b) reaction of a compound of structure (III)

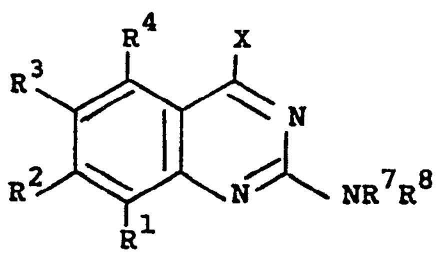

(III)

in which $R^1$ to $R^4$ and $R^7$ and $R^8$ are as described for structure (I) and X is a group displaceable by an amine, with an amine of structure $R^5R^6NH$ in which $R^5$ and $R^6$ are as described for structure (I) ; and optionally thereafter,

° removing any protecting groups ;

° forming a pharmaceutically acceptable salt.

Suitable groups displaceable by an amine, X, will be apparent to those skilled in the art and include, for example, halogen, in particular chlorine, $SC_{1-4}$alkyl, such as methylthio, hydroxy and phenoxy.

Reaction of a compound of structure (II) with an amine $R^7R^8NH$ is suitably carried out in an inert solvent at elevated temperature. Preferably the reaction is carried out in the absence of a solvent in a sealed receptacle at elevated temperature.

Reaction of a compound of structure (III) with an amine $R^5R^6NH$ is suitably carried out in the presence or absence of an inert solvent at elevated temperature. Suitable solvents include, for example $C_{1-4}$alkanols such as isopropanol or butanol, preferably isopropanol.

In particular, leaving groups X and $X^1$ are halogen, preferably chlorine, and can be displaced by appropriate amines $R^5R^6NH$ and $R^7R^8NH$ under the general conditions described above and in the specific examples. Other conditions and reagents depending on the nature of the leaving groups will be apparent to those skilled in the art ; for example compounds of structure (I) in which $R^5$ and $R^6$ are both hydrogen, can be prepared from the corresponding compounds of structure (III) in which X is hydroxy by reaction with phenylphosphordiamidate using the method described in J. Het. Chem (1972), 9, 1235.

Pharmaceutically acceptable acid addition salts of the compounds of structure (I) can be prepared by standard procedures by, for example, reaction with suitable organic and inorganic acids the nature of which will be apparent to persons skilled in the art. For example, pharmaceutically acceptable salts can be formed by reaction with hydrochloric, sulphuric, or phosphoric acids ; aliphatic, aromatic or heterocyclic sulphonic acids or carboxylic acids such as, for example, citric, maleic or fumaric acids.

The intermediate compounds of structure (II) and (III) can be prepared by procedures analogous to those known in the art. The amines of structure $R^5R^6NH$ and $R^7R^8NH$ are available commercially or can be prepared by standard techniques well known to those skilled in the art of organic chemistry.

For example compounds of structure (II) in which $R^1$ is $OCH_3$, $R^2$ to $R^4$ are all hydrogen and X is chlorine can be prepared by the route outlined in Scheme I. It will be apparent to those skilled in the art that the reactions of Scheme 1 can also be carried out on compounds (A) in which $R^1$ to $R^4$ have different values to those indicated to produce further compounds of structure (II).

<u>Scheme I</u>

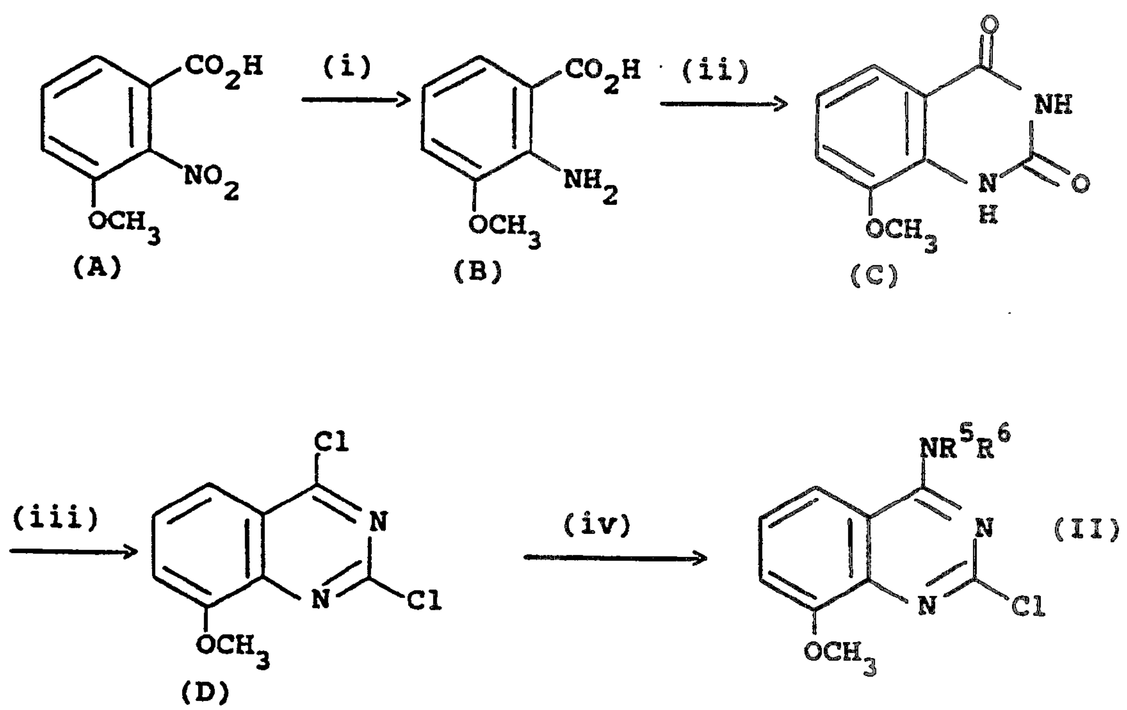


(A) (B) (C) (D) (II)

(i) $H_2$, Pd/C 10%, EtOH
(ii) KNCO, AcOH, NaOH
(iii) $POCl_3$, $PhNMe_2$, Δ
(iv) $R^5R^6NH$, NaOAc, $THF/H_2O$, Δ.

Compounds of structure (III) in which $R^1$ to $R^4$ are all hydrogen and $R^7$ and $R^8$ are both hydrogen, $C_{1-4}$alkyl

or $(CH_2)_nAr^1$ or one is $C_{1-4}$alkyl and the other is $(CH_2)_nAr^1$ and X is chlorine can be prepared by the procedures outlined in Scheme II. Again, it will be apparent to those skilled in the art that the reactions of Scheme (II) can be carried out on compounds (E) in which $R^1$ to $R^4$ are other than all hydrogen to product further compounds of structure (III).

Scheme II

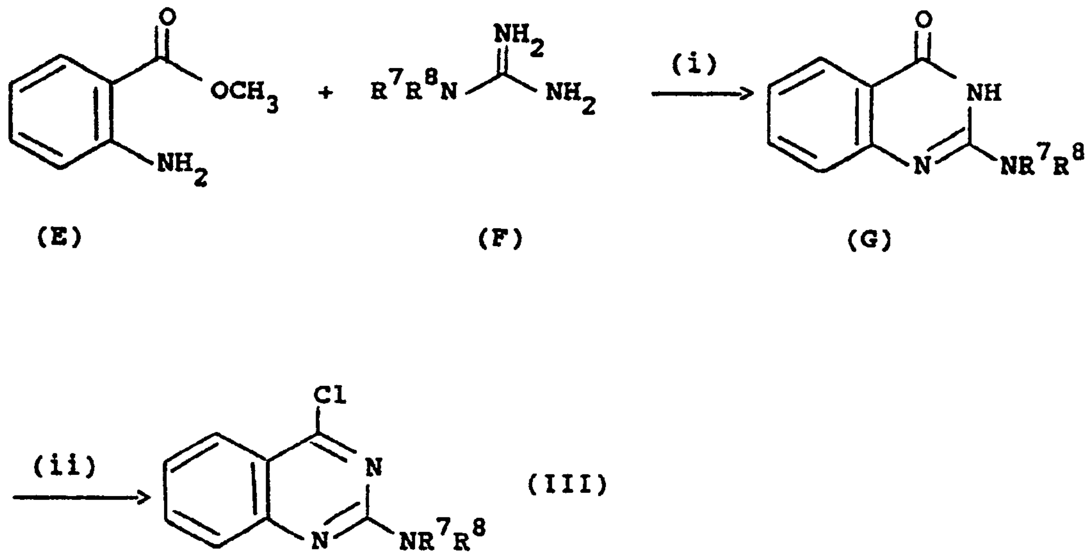


(E) (F) (G)

(III)

(i) $NaOCH_3$, nBuOH
(ii) $POCl_3$

Alternatively, compounds of structure (III) can be prepared from compounds of structure (D) – see Scheme I – by the method used in J. Med. Chem., 1981, 24, 127-140. This alternative method is outlined in Scheme III.

Scheme III

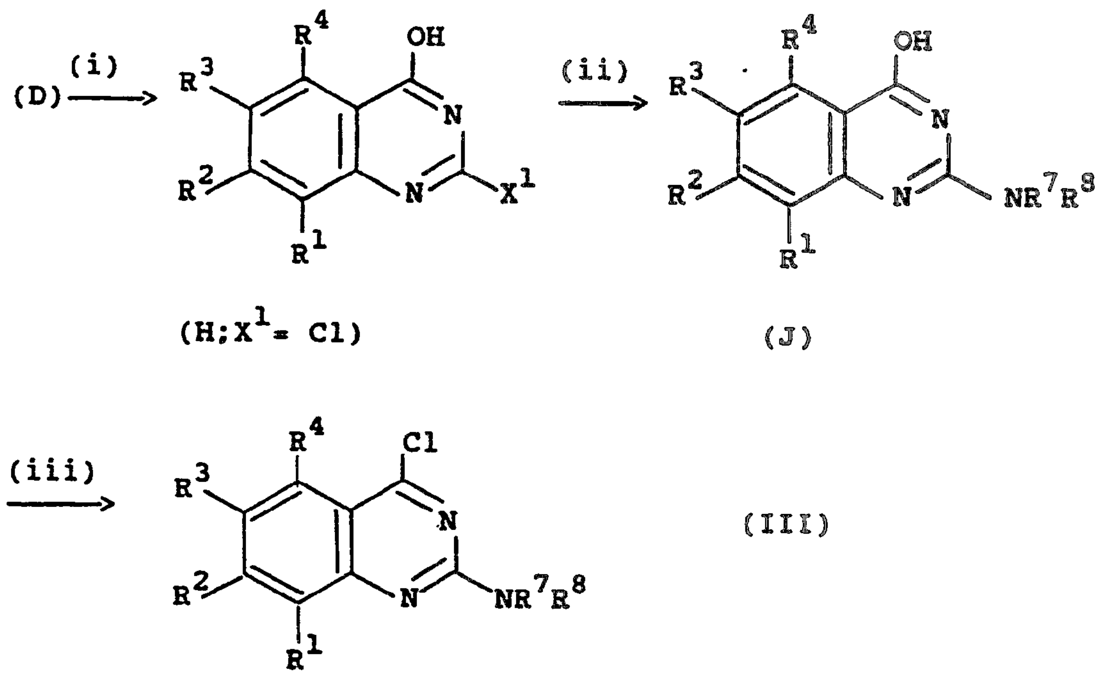


(H; $X^1$ = Cl)

(J)

(III)

(i) NaOH/$H_2O$
(ii) $R^7R^8NH$, solvent, Δ
(iii) $POCl_3$, solvent, Δ

The starting materials used to prepare compounds of structures (II) and (III) are available commercially or can be prepared by standard techniques. In addition it will be appreciated that further variations of the above-noted schemes can be utilized, for example, in Scheme III, compounds of structure (H) in which $X^1$ is Cl can be replaced by compounds of structure (H) in which $X^1$ is, for example methylthio and then converted to compounds of structure (III) as shown. Such compounds in which $X^1$ is methylthio are prepared by standard techniques for example by treatment of the analogous thione precursor with methyl iodide in ethanol in the presence of sodium hydroxide ; or, for example, when $R^1$ to $R^4$ are all H, are commercially available.

It is to be noted, and apparent to those skilled in the art that in the foregoing reactions, where necessary groups $R^1$ to $R^4$ and groups on aromatic rings Ar and $Ar^1$ (e.g. hydroxy or amino groups) will be in "protected" form. For example, amino groups can be "protected" in the form of nitro groups and converted into amino groups as appropriate, and hydroxy groups can be protected using standard groups for example as described in "Greene, T.W., Protective Groups in Organic Chemistry" which also provides examples of further appropriate protective groups for other moities.

The compounds of structure (I) and their pharmaceutically acceptable salts exert an anti-secretory effect by inhibition of the gastrointestinal $H^+K^+$ATPase enzyme (Fellenius E., Berglindh T., Sachs G., Olke L., Elander B., Sjostrand S.E., and Wallmark B., 1981, Nature, 290, 159-61).

In a further aspect therefore the present invention provides compounds of structure (I) and pharmaceutically acceptable salts thereof for use in therapy.

The compounds of structure (I) and their pharmaceutically acceptable salts inhibit exogenously and endogenously stimulated gastric acid secretion and are useful in the treatment of gastrointestinal diseases in mammals, in particular humans. Such diseases include, for example, gastric and duodenal ulcers, and Zollinger-Ellison Syndrome. Further, the compounds of structure (I) can be used in the treatment of other disorders where an anti-secretory effect is desirable for example in patients with gastritis, NSAID induced gastritis, gastric ulcers, acute upper intestinal bleeding, in patients with a history of chronic and excessive alcohol consumption, and in patients with gastro oesophageal reflux disease (GERD).

In therapeutic use, the compounds of the present invention are usually administered in a standard pharmaceutical composition. The present invention therefore provides in a further aspect pharmaceutical compositions comprising a compound of structure (I) or a pharmaceutically acceptable salt thereof and a pharmaceutically acceptable carrier.

The compounds of structure (I) and their pharmaceutically acceptable salts which are active when given orally can be formulated as liquids, for example syrups, suspensions or emulsions, tablets, capsules and lozenges.

A liquid formulation will generally consist of a suspension or solution of the compound or pharmaceutically acceptable salt in a suitable liquid carrier(s) for example, ethanol, glycerine, non-aqueous solvent, for example polyethylene glycol, oils, or water with a suspending agent, preservative, flavouring or colouring agent.

A composition in the form of a tablet can be prepared using any suitable pharmaceutical carrier(s) routinely used for preparing solid formulations. Examples of such carriers include magnesium stearate, starch, lactose, sucrose and cellulose.

A composition in the form of a capsule can be prepared using routine encapsulation procedures. For example, pellets containing the active ingredient can be prepared using standard carriers and then filled into a hard gelatin capsule ; alternatively, a dispersion or suspension can be prepared using any suitable pharmaceutical carrier(s), for example aqueous gums, celluloses, silicates or oils and the dispersion or suspension then filled into a soft gelatin capsule.

Typical parenteral compositions consist of a solution or suspension of the compound or pharmaceutically acceptable salt in a sterile aqueous carrier or parenterally acceptable oil, for example polyethylene glycol, polyvinyl pyrrolidine, lecithin, arachis oil or sesame oil. Alternatively, the solution can be lyophilised and then reconstituted with a suitable solvent just prior to administration.

A typical suppository formulation comprises a compound of formula (I) or a pharmaceutically acceptable salt thereof which is active when administered in this way, with a binding and/or lubricating agent such as polymeric glycols, gelatins or cocoa butter or other low melting vegetable or synthetic waxes or fats.

Preferably the composition is in unit dose form such as a tablet or capsule.

Each dosage unit for oral administration contains preferably from 1 to 250 mg (and for parenteral administration contains preferably from 0.1 to 25 mg) of a compound of the formula (I) or a pharmaceutically acceptable salt thereof calculated as the free base.

The present invention also provides a method of inhibiting gastric acid secretion which comprises administering to a mammal in need thereof an effective amount of a compound of structure (I) or a pharmaceutically acceptable salt thereof ; and a method of treatment of diseases of the stomach or intestine based on increased acid secretion which comprises administering to a mammal in need thereof an effective amount of a compound of structure (I) or a pharmaceutically acceptable salt thereof.

The pharmaceutically acceptable compounds of the invention will normally be administered to a subject for the treatment of gastrointestinal diseases and other conditions caused or exacerbated by gastric acidity. The daily dosage regimen for an adult patient may be, for example, an oral dose of between 1 mg and 500 mg, preferably between 1 mg and 250 mg, or an intravenous, subcutaneous, or intramuscular dose of between 0.1 mg and 100 mg, preferably between 0.1 mg and 25 mg, of the compound of the formula (I) or a pharmaceutically acceptable salt thereof calculated as the free base, the compound being administered 1 to 4 times per day. Suitably the compounds will be administered for a period of continuous therapy, for example for a week or more.

In addition, the compounds of the present invention can be co-administered with further active ingredients, such as antacids (for example magnesium carbonate or hydroxide and aluminium hydroxide), non-steroidal anti-flammatory drugs (for example indomethacin, aspirin or naproxen), steroids, or nitrite scavengers (for example ascorbic acid or aminosulphonic acid), or other drugs used for treating gastric ulcers (for example pirenzipine, prostanoids for example 16,16 dimethyl $PGE_2$, or histamine $H_2$-antagonists (for example, cimetidine).

The following examples illustrate the invention. Temperatures are recorded in degrees centigrade.

## Example 1

### Preparation of 2-amino-8-methoxy-4-(2-methylphenylamino)quinazoline

A. Preparation of 3-methoxyanthranilic acid

2-Nitro-3-methoxybenzoic acid (10 g, 0.051 mol) was suspended in ethanol with palladium/charcoal 10% (1 g). The mixture was placed under hydrogen (50 psi) and shaken on a Parr until theoretical uptake had been achieved. The suspension was then flushed with nitrogen, the charcoal filtered off, and the filtrate evaporated in vacuo to give 3-methoxyanthranilic acid (7.76 g, 91%) m.p. 172-175°C.

B. Preparation of 8-methoxy-2,4-quinazolinedione

3-Methoxyanthranilic acid (6 g, 0.036 mol) was suspended in water (200 ml, 35°C) and glacial acetic acid (2.2 ml). A freshly prepared solution of potassium cyanate (3.7 g, 0.046 mol) in water (20 ml) was added dropwise to the stirred mixture. After 2 hours, sodium hydroxide (48.5 g 1.21 mol) was added in portions keeping the temperature below 40°C. A clear solution was obtained momentarily before precipitating the hydrated sodium salt. After cooling, the precipitate was filtered off, dissolved in hot water which was acidified to pH 5, causing precipitation of 8-methoxy-2,4-quinazolinedione (4.6 g, 58%) m.p. 255-257°C.

C. Preparation of 8-methoxy-2,4-dichloroquinazoline

A suspension of 8-methoxy-2,4-quinazolinedione (4 g, 0.019 mol) suspended in phosphoryl chloride (10 ml 0.108 mol) and N,N-dimethylaniline (1.6 ml, 0.0125 mol) was heated under reflux for 5 hours. The reaction mixture was poured onto ice and the precipitate washed and dried to give 8-methoxy-2,4-dichloroquinazoline (3.79 g, 87%) m.p. 155-157°C.

D. Preparation of 8-methoxy-4-(2-methylphenylamino)-2-chloroquinazoline

8-Methoxy-2,4-dichloroquinazoline (3.7 g, 0.016 mol) was stirred in a mixture of water (85 ml), tetrahydrofuran (125 ml), o-toluidine (1.7 g, 0.016 mol) and sodium acetate (2.2 g, 0.027 mol) for a total of 4 days with heating to 50°C for a total of 32 hours and the addition of a total of 20 ml 0.01 mol NaOH dropwise over this period maintaining the pH at 7. The reaction mixture was evaporated in vacuo and crystallized from ethanol/water to give 8-methoxy-4-(2-methylphenylamino)-2-chloroquinazoline (2.89 g, 60%) m.p. 218-220°C.

E. Preparation of 2-amino-4-(2-methylphenylamino)-8-methoxyquinazoline

8-Methoxy-4-(2-methylphenylamino)-2-chloroquinazoline (1.8 g, 0.006 mol) was dissolved in ethanolic ammonia and heated in a sealed vessel at 120°C for 3 hours. After cooling, removal of excess solvent and chromatography (silica gel, 2% methanolic ammonia in chloroform) the 2-amino-4-(2-methylphenylamino)-8-methoxyquinazoline was isolated as crystals (0.52 g, 31%) from ethanol, m.p. 218-220°C.

| | $C_{16}H_{16}N_4O$ 0.37EtOH |
|---|---|
| Found | C 67.61, H 6.26, N 18.81 |
| Requires | C 67.61, H 6.17, N 18.84 |

Example 2

Preparation of 2-amino-8-methoxy-4-benzylaminoquinazoline

A. Preparation of 2-chloro-8-methoxy-4-benzylaminoquinazoline

Substituting benzylamine (1.4 g) for o-toluidine and using corresponding molar proportions in Example 1D gave, 2-chloro-8-methoxy-4-benzylaminoquinazoline (3.2 g, 82%) m.p. 253-254°C.

B. Preparation of 2-amino-8-methoxy-4-benzylaminoquinazoline

Substituting 2-chloro-8-methoxy-4-benzylaminoquinazoline (2 g, 0.0067 mol) for 2-chloro-8-methoxy-4-(2-methylphenylamino)quinazoline and using corresponding molar proportions of the other reagents in Example 1E, gave 2-amino-8-methoxy-4-benzylaminoquinazoline (0.29 g, 15%) from ethanol, m.p. 243-245°C.

| | $C_{16}H_{16}N_4O$ |
|---|---|
| Found | C 68.77, H 5.52, N 20.16 |
| Requires | C 68.55, H 5.75, N 19.99 |

Example 3

Preparation of 2-amino-8-methoxy-4-(2-methyl-4-methoxyphenylamino)quinazoline

A. Preparation of 2-chloro-8-methoxy-4-(2-methyl-4-methoxyphenylamino)quinazoline

Substituting 4-methoxy-o-toluidine (1.6 g, 0.0122 mol) for o-toluidine and using corresponding molar proportions of the other reagents in Example 1D, gave 2-chloro-8-methoxy-4-(4-methoxy-2-methylphenylamino)quinazoline (2.02 g, 51%) m.p. 230-232°C.

B. Preparation of 2-amino-8-methoxy-4-(2-methyl-4-methoxyphenylamino)quinazoline

Substituting 2-chloro-8-methoxy-4-(2-methyl-4-methoxyphenylamino)quinazoline (2.0 g, 0.006 mol) for 2-chloro-8-methoxy-4-(2-methylphenylamino)quinazoline and using corresponding molar proportions of the other reagents in the Example 1E, gave 2-amino-8-methoxy-4-(2-methyl-4-methoxyphenylamino)quinazoline (0.25 g, 13%) from ethanol/water, m.p. 185-187°C.

| | $C_{17}H_{18}N_4O_2$ 0.5 $H_2O$ |
|---|---|
| Found | C 63.95, H 5.77, N 17.52 |
| Requires | C 63.93, H 5.99, N 17.54 |

Example 4

Preparation of 2-amino-8-methoxy-4-(2-methylbenzylamino)quinazoline

A. Preparation of 2-chloro-8-methoxy-4-(2-methylbenzylamino)quinazoline

Substituting 2-methylbenzylamine for o-toluidine (1.41 g, 0.0117 mol) and using corresponding molar proportions of the other reagents in the Example 1D, gave 2-chloro-8-methoxy-4-(2-methylbenzylamino)quinazoline (3.36 g, 89%) m.p. 279-281°C.

B. Preparation of 2-amino-8-methoxy-4-(2-methylbenzylamino)quinazoline

Substituting 2-chloro-8-methoxy-4-(2-methylbenzylamino)quinazoline (2.3 g, 0.0073 mol) for 2-chloro-8-methoxy-4-(2-methylphenylamino)quinazoline in Example 1E, gave 2-amino-8-methoxy-4-(2-methylbenzylamino)quinazoline (0.4 g, 18.5%) from acetonitrile, m.p. 274-275°C.

| | $C_{17}H_{18}N_4O$ 0.67$H_2O$ 0.36$CH_3CN$ |
|---|---|
| Found | C 68.78, H 6.20, N, 19.07 |
| Requires | C 69.03, N 6.19, N, 19.03 |

Example 5

Preparation of 2-dimethylamino-8-methoxy-4-(2-methylphenylamino)quinazoline

A. Preparation of 2-dimethylamino-8-methoxy-4-(2-methylphenylamino)quinazoline

Substituting ethanolic dimethylamine for ethanolic ammonia and using corresponding molar proportions of the other reagents in the Example 1E, gave 2-dimethylamino-8-methoxy-4-(2-methylphenylamino)quinazoline (0.73 g, 59%) from acetonitrile, m.p. 141-143°C.

| | $C_{18}H_{20}N_4O\ 0.2H_2O$ |
|---|---|
| Found | C 69.37, H 6.34, N 18.01 |
| Requires | C 69.30, H 6.59, N 17.96 |

Example 6

Preparation of 2-amino-8-methyl-4-(2-methylphenylamino)quinazoline

A. Preparation of 8-methyl-2,4-quinazolone

Substituting 3-methylanthranilic acid (5 g, 0.033 mol) for 3-methoxyanthranilic acid and using corresponding molar proportions of the other reagents in Example 1B gave 8-methyl-2,4-quinazolinedione (4.18 g, 72%), m.p. 285-287°C.

B. Preparation of 2,4-dichloro-8-methylquinazoline

Substituting 8-methyl-2,4-quinazolinedione (4.0 g, 0.023 mol) for 8-methoxy-2,4-quinazolinedione and using corresponding molar proportions of the other reagents in Example 1C, gave 2,4-dichloro-8-methylquinazoline (4.12 g, 84%), m.p. 180-230°C which was used without further purification.

C. Preparation of 2-chloro-8-methyl-4-(2-methylphenylamino)quinazoline

Substituting 2,4-dichloro-8-methyl-4-(2-methylphenylamino)quinazoline (3.7 g, 0.018 mol) for 2,4-dichloro-8-methoxyquinazoline and using corresponding molar proportions of the other reagents in Example 1D, gave 2-chloro-8-methyl-4-(2-methylphenylamino)quinazoline (3.96 g, 77%), m.p. 125-126°C.

D. Preparation of 2-amino-8-methyl-4-(2-methylphenylamino)quinazoline

Substituting 2-chloro-8-methyl-4-(2-methylphenylamino)quinazoline (2.0 g, 0.007 mol) for 2-chloro-8-methoxy-4-(2-methylphenylamino)quinazoline and using corresponding molar proportions of the other reagents in Example 1E, gave 2-amino-8-methyl-4-(2-methylphenylamino)quinazoline (0.27 g, 15.3%) from acetonitrile/water, m.p. 118-120°C.

| | $C_{16}H_{16}N_4$ |
|---|---|
| Found | C 72.59, H 6.21, N 21.17 |
| Requires | C 72.70, H 6.10, N 21.20 |

Example 7

Preparation of 2-amino-4-(2-methylphenylamino)quinazoline

A. Preparation of 2-amino-4-(3H)-quinazolone

Guanidine hydrochloride (47.77 g, 0.5 mol) was added portionwise to a stirred suspension of sodium methoxide (32.42 g, 0.60 mol) in n-butanol (450 ml) at ambient temperature over 0.5 hour. After a further 0.5 hour a solution of methyl anthranilate (15 g, 0.10 mol) in n-butanol (150 ml) was added dropwise and then slowly brought to reflux. After distilling off ca. 100 ml of solvent the reaction mixture was heated under reflux at 116°C for 117 hours. The cooled suspension was filtered, excess solvent removed and the residue dissolved in water, acidified to pH 5 and extracted with diethyl ether. The aqueous suspension was reacidified to pH 5, filtered off, washed with water and dried. The solid was then triturated in methanol, filtered, washed with ether and dried to give 2-amino-4-(3H)-quinazolone (3.0 g, 19%) m.p. >300°C.

B. Preparation of 2-amino-4-chloroquinazoline

2-Amino-4-(3H)-quinazolone (2.0 g, 0.0124 mol) was heated under reflux in phosphoryl chloride (19.02 g, 0.0124 mol) for 2.5 hours. The reaction mixture was partitioned between chloroform, ice and NaOH solution (pH 9) and the organic phase dried, filtered, excess solvent removed and the residue triturated with chloroform to give 2-amino-4-chloroquinazoline (0.42 g, 19%) m.p. 275-278°C.

C. Preparation of 2-amino-4-(2-methylphenylamino)quinazoline

2-Amino-4-chloroquinazoline (0.64 g, 0.0036 mol) was heated to 170°C in o-toluidine (1 ml, 0.008 mol) for 1 hour. The reaction mixture was dissolved in ethanol, stripped and partitioned between chloroform and NaOH solution (pH 9). The organic solution was dried, filtered and excess solvent removed to give an oil which afforded crystals of 2-amino-4-(2-methylphenylamino)quinazoline (0.55 g, 62%) m.p. 273-275°C (from ethanol).

| | $C_{15}H_{14}N_4$ 0.02$CHCl_3$ |
|---|---|
| Found | C 71.08, H 5.42, N 22.04 |
| Requires | C 71.36, H 5.59, N 22.16 |

Example 8

Preparation of 2-pyrrolidino-4-(2-methylphenylamino)quinazoline

A. Preparation of 2-chloro-4-(2-methylphenylamino)quinazoline

Substituting 2,4-dichloroquinazoline (9.95 g, 0.05 mol) for 8-methoxy-2,4-dichloroquinazoline and using corresponding molar proportions of the other reagents in Example 1D, gave 2-chloro-4-(2-methylphenylamino)quinazoline (10.19 g, 76%) m.p.192-194°C.

B. Preparation of 2-pyrrolidino-4-(2-methylphenylamino)quinazoline

2-chloro-4-(2-methylphenylamino)quinazoline (5 g, 0.0185 mol) and pyrrolidine (6.59 g, 0.093 mol) were dissolved in ethanol (65 ml) placed in a sealed vessel and heated to 135°C for 5 hours. After cooling, the reaction mixture was dissolved in ethanol and then evaporated in vacuo. The oily residue afforded crystals of 2-pyrrolidino-4-(2-methylphenylamino)quinazoline (3.67 g, 65%) from ethanol/water, m.p. 152-154°C.

| | $C_{19}H_{20}N_4$ |
|---|---|
| Found | C 74.58, H 6.60, N 18.49 |
| Requires | C 74.97, H 6.62, N 18.41 |

Example 9

Preparation of 2-ethylamino-4-(2-methylphenylamino)quinazoline

2-Chloro-4-(2-methylphenylaminomethyl)quinazoline (5.00 g, 0.0185 mol) and ethylamine in ethanol (33%, 30 ml) were dissolved in ethanol (35 ml) placed in a sealed vessel and heated for 5 hours at 130°C. After cooling and removal of excess solvent in vacuo the residue afforded crystals of 2-ethylamino-4-(2-methylphenylamino)quinazoline (1.7 g, 33%) from ethanol/water, m.p. 127-129°C.

| | $C_{17}H_{18}N_4$ |
|---|---|
| Found | C 73.09, H 6.43, N 20.06 |
| Requires | C 73.35, H 6.52, N 20.13 |

Example 10

Preparation of 2-benzylamino-4-(2-methylphenylamino)quinazoline

2-Chloro-4-(2-methylphenylamino)quinazoline (2.7 g, 0.01 mol) and benzylamine (2,4 g, 0.022 mol) were dissolved in n-butanol (20 ml) and heated under reflux for 5 hours. The solution was cooled, excess solvent removed in vacuo and the residue treated with water, filtered and crystallised from ethanol/water. The compound was then chromatographed (silica gel, 0.5% methanolic ammonia/chloroform) to afford an oil, which on trituration with ether gave crystals of 2-benzylamino-4-(2-methylphenylamino)quinazoline (1.88 g, 55%), m.p. 127-130°C.

| | $C_{22}H_{20}N_4$ |
|---|---|
| Found | C 77.50, H 6.00, N 16.45 |
| Requires | C 77.62, H 5.92, N 16.46 |

Example 11

Preparation of 2-amino-4-(2-methylphenylamino)-6-ethylquinazoline

A. Preparation of 6-ethyl-2,4-quinazoline dione

Substituting 6-ethylanthranilic acid for 3-methoxyanthranilic acid and using corresponding molar proportions of the other reagents in Example 1B, gave 6-ethyl-2,4-quinazoline dione (20.15 g, 90%) m.p. 325°C.

B. Preparation of 6-ethyl-2,4-dichloroquinazoline

Substituting 6-ethyl-2,4-quinazolinedione (20 g, 0.105 mol) for 8-methoxy-2,4-quinazolinedione and using corresponding molar proportions of the other reagents in Example 1C, gave 6-ethyl-2,4-dichloroquinazoline (19.57 g, 82%) m.p. 90-92°C.

C. Preparation of 2-chloro-4-(2-methylphenylamino)-6-ethylquinazoline

Substituting 6-ethyl-2,4-dichloroquinazoline (10.0 g, 0.044 mol) for 8-methoxy-2,4-dichloroquinazoline and using corresponding molar proportions of the other reagents in Example 1D, gave 2-chloro-4-(2-methylphenylamino)-6-ethylquinazoline (7.23 g, 56%) m.p. 177-179°C.

D. Preparation of 2-amino-4-(2-methylphenylamino)-6-ethylquinazoline

Substituting 2-chloro-4-(2-methylphenylamino)-6-ethylquinazoline (2.98 g, 0.01 mol) for 8-methoxy-2-chloro-4-(2-methylphenylamino)quinazoline and using corresponding molar proportions of the other reagents in Example 1E, gave 2-amino-4-(2-methylphenylamino)-6-ethylquinazoline (0.76 g, 27%) from ether, m.p. 263-265°C.

| | $C_{17}H_{18}N_4$ 1.5% w/w $CHCl_3$ |
|---|---|
| Found | C 72.18, H 6.49, N 19.76 |
| Requires | C 72.40, H 6.43, N 19.83 |

Example 12

Preparation of 8-methoxy-2,4-bis(2-methylphenylamino)quinazoline

8-methoxy-4-(2-methylphenylamino)-2-chloroquinazoline (1.00 g, 0.0033 mol) was dissolved in ethanol (15 ml) with o-toluidine (1.06 g, 0.0099 mol) and heated in a sealed vessel at 150°C for 5 hours. After cooling and removal of excess solvent in vacuo the solid was chromatographed (silica gel, chloroform). 8-Methoxy-2,4-bis(2-methylphenylamino)quinazoline was isolated as crystals (0.93 g, 76%) from ethanol/water, m.p. 185-187°C.

| | $C_{23}H_{22}N_4O$ |
|---|---|
| Found | C 74.48, H 6.00, N 14.92 |
| Requires | C 74.57, H 5.99, N 15.12 |

Example 13

Preparation of 2-methylamino-8-methoxy-4-(2-methylphenylamino)quinazoline

Substituting ethanolic methylamine for ethanolic ammonia and using corresponding molar proportions of the other reagents in the Example 1E, gave 2-methylamino-8-methoxy-4-(2-methylphenylamino)quinazoline (0.3 g, 35%) from ethanol/water m.p. 190-192°C.

| | $C_{17}H_{18}N_4O$ |
|---|---|
| Found | C 69.24, H 6.07, N 18.81 |
| Requires | C 69.37, H 6.16, N 19.04 |

Example 14

Preparation of 2-benzylamino-8-methoxy-4-(2-methylphenylamino)quinazoline

Substituting benzylamine (0.53 g 0.00495 mol) for o-toluidine and using corresponding molar proportions of the other reagents in Example 12 gave 2-benzylamino-8-methoxy-4-(2-methylphenylamino)quinazoline hydrochloride (0.62 g, 46%) from ethanolic HCl, m.p. 237-238°C.

| | $C_{23}H_{22}N_4O.HCl\ 0.5H_2O$ |
|---|---|
| Found | C 65.65, H 5.69, N 13.41, Cl 8.3 |
| Requires | C 67.42, H 5.81, N 13.47, Cl 8.52 |

Example 15

Preparation of 2-pyrrolidino-8-methoxy-4-(2-methylphenylamino)quinazoline

Substituting pyrrolidine (2.1 g, 0.03 mol) for o-toluidine and using corresponding molar proportions of the other reagents in Example 12 gave 2-pyrrolidino-8-methoxy-4-(2-methylphenylamino)quinazoline (1.71 g, 77%) from ethanol, m.p. 181-183°C.

| | $C_{20}H_{22}N_4O$ |
|---|---|
| Found | C 71.55, H 6.75, N 16.60 |
| Requires | C 71.83, H 6.63, N 16.75 |

Example 16

Preparation of 2-phenylethylamino-4-(2-methylphenylamino)quinazoline

Substituting phenylethylamine (3.64 g, 0.03 mol) for benzylamine and using corresponding molar proportions of the other reagents in Example 10 and using ethanol as the solvent gave 2-phenylethylamino-4-(2-methylphenylamino)quinazoline (1.45 g, 58%) from ether, m.p. 95-96°C.

| | $C_{23}H_{22}N_4O.15H_2O$ |
|---|---|
| Found | C 77.39, H 6.24, N 15.71 |
| Requires | C 77.34, H 6.29, N 15.68 |

Example 17

Preparation of 8-methyl-4-(2-methylphenylamino)-2-(2-phenylethylamino)quinazoline hydrochloride

8-Methyl-4-(2-methylphenylamino)-2-chloroquinazoline (0.71 g, 0.0025 mol) was dissolved in ethanol (20 ml) with 2-phenylethylamine (0.61 g, 0.005 mol) and heated in a sealed vessel at 140°C for 4 hours. After cooling and removal of excess solvent in vacuo the glass was dissolved in a small amount of methanol and 2N HCl added to form the hydrochloride salt which was recrystallized from ethanol/ether to give 8-methyl-4-(2-methylphenylamino)- 2-(2-phenylethylamino)quinazoline hydrochloride (0.5 g, 50%), m.p. >250°C.

| | $C_{24}H_{24}N_4\ 1.0HCl$ |
|---|---|
| Found | C 71.28, H 6.35, N 13.82, Cl 8.77 |
| Requires | C 71.18, H 6.22, N 13.84, Cl 8.76 |

Example 18

Preparation of 2-amino-8-methoxy-4-(2-methoxyphenylamino)quinazoline

A. Preparation of 2-chloro-8-methoxy-4-(2-methoxyphenylamino)quinazoline

Substituting o-anisidine (2.94 g) for o-toluidine and using corresponding molar proportions of the other reagents in Example 1D gave, 2-chloro-8-methoxy-4-(2-methoxyphenylamino)quinazoline (6.74 g, 98%) m.p. 194-196°C.

B. Preparation of 2-amino-8-methoxy-4-(2-methoxyphenylamino)quinazoline

Substituting 2-chloro-8-methoxy-4-(2-methoxyphenylamino)quinazoline (4.0 g) for 2-chloro-8-methoxy-4-(2-methylphenylamino)quinazoline and using corresponding molar proportions of the other reagents in Example 1E gave, after recrystallisation from ethanol/water 2-amino-8-methoxy-4-(2-methoxyphenylamino)quinazoline (0.16 g, 4.2%), m.p. 243-244°C.

| | $C_{16}H_{16}N_4O_2$ 0.18 $H_2O$ |
|---|---|
| Found | C 63.88, H 5.38, N 18.87 |
| Requires | C 64.14, H 5.50, N 18.70. |

Example 19

Preparation of 2,4-Bis-(N-methylphenylamino)-8-methoxyquinazoline

8-Methoxy-2,4-dichloroquinazoline (1.5 g, 0.007 mol) was heated under reflux in a solution of N-methyl aniline (1.43 ml, 0.014 mol) in tetrahydrofuran (50 ml) for 16 hours precipitating a solid, which was collected and crystallised from ethanol/water to give 2,4-bis-(N-methylphenylamino)-8-methoxyquinazoline (0.37 g, 15%), m.p. 169-170°C.

| | $C_{23}H_{22}N_4O$ |
|---|---|
| Found | C 74.32, H 5.85, N 15.04. |
| Requires | C 74.57, H 5.99, N 15.12. |

Example 20

Preparation of 2,4-Bis-(N-methylphenylamino)quinazoline hydrochloride

Substituting 2,4-dichloroquinazoline (4.5 g, 0.0226 mol) for 8-methoxy-2,4-dichloroquinazoline and using corresponding molar proportions of the other reagents in Example 20, gave after crystallisation from ethanolic hydrogen chloride 2,4-bis-(N-methylphenylamino)quinazoline hydrochloride (2.2 g, 30%), m.p. 243-244°C.

| | $C_{22}H_{20}N_4$ 1.0HCl |
|---|---|
| Found | C 69.79, H 5.56, N 14.72, Cl 9.02 |
| Requires | C 69.89, H 5.70, N 14.68, Cl 9.29 |

Example 21

Preparation of 2-(N-methylphenylamino)-4-(2-methylphenylamino)-8-methoxyquinazoline

Substituting N-methylaniline (1.4 g, 0.013 mol) for o-toluidine and using the corresponding molar proportions of the other reagents in Example 12 gave after crystallisation from ethanol 2-(N-methylphenylamino)-4-(2-methylphenylamino)quinazoline (0.45 g, 45%), m.p. 145-147°C.

| | $C_{23}H_{22}N_4O$ |
|---|---|
| Found | C 74.89, H 6.10, N 15.34. |
| Requires | C 74.57, H 5.99, N 15.12. |

Example 22

Preparation of 2-(N-methylphenylamino)-4-(2-methylphenylamino)quinazoline hydrochloride

Substituting N-methylaniline (2.39 g, 0.022 mol) for benzylamine and using corresponding molar proportions of the other reagents in Example 10 and using ethanol as the solvent gave after crystallisation from ethanolic hydrogen chloride 2-(N-methylphenylamino)-4-(2-methylphenylamino)quinazoline hydrochloride (1.64 g, 39%), m.p. 284-286°C.

| | $C_{22}H_{20}N_4$ 1.0HCl |
|---|---|
| Found | C 70.06, H 5.73, N 14.83, Cl 9.17 |
| Requires | C 70.11, H 5.62, N 14.87, Cl 9.41 |

Example 23

Preparation of 2-phenethylamino-4-(2-methylphenylamino)-8-methoxyquinazoline hydrochloride

Substituting phenylethylamine (1.2 ml, 0.009 mol) for o-toluidine and using corresponding molar proportions of the other reagents in Example 12, gave after crystallisation from ethanolic hydrogen chloride/ether 2-phenethylamino-4-(2-methylphenylamino)-8-methoxyquinazolinehydrochloride (0.38 g, 42%), m.p. 263-265°C.

| | $C_{24}H_{24}N_4O$ 1.0HCl |
|---|---|
| Found | C 68.62, H 6.06, N 13.64, Cl 8.22 |
| Requires | C 68.48, H 5.99, N 13.31, Cl 8.42 |

Example 24

Preparation of 4-(N-methylphenylamino)-2-(2-methylphenylamino)-8-methoxyquinazoline

A. Preparation of 8-methoxy-4-(N-methylphenylamino)-2-chloroquinazoline

Substituting N-methylaniline (3.85 g, 0.036 mol) for o-toluidine and using corresponding molar proportions of the other reagents in Example 1D, gave after crystallisation from ethanol/water 8-methoxy-4-(N-methylphenylamino)-2-chloroquinazoline (6.19 g, 63%), m.p. 115-117°C.

B. Preparation of 8-methoxy-4-(N-methylphenylamino)-2-(2-methylphenylamino)quinazoline hydrochloride

Substituting 8-methoxy-4-(N-methylphenylamino)-2-chloroquinazoline (1.5 g, 0.005 mol) for 8-methoxy-4-(2-methylphenylamino)-2-chloroquinazoline and using corresponding molar proportions of the other reagents in Example 12, gave after crystallisation from ethanolic hydrogen chloride/ether 4-(N-methylphenylamino)-2-(2-methylphenylamino)-8-methoxyquinazoline hydrochloride (0.99 g, 49%). m.p. 232-234°C.

| | $C_{23}H_{22}N_4O$ 1.0HCl |
|---|---|
| Found | C 67.74, H 5.63, N 13.76, Cl 8.39 |
| Requires | C 67.89, H 5.70, H 13.77, Cl, 8.71 |

Example 25

Preparation of 2-amino-4-(2-methylbenzylamino)quinazoline

A. Preparation of 2-chloro-4-(2-methylbenzylamino)quinazoline

2,4-Dichloroquinazoline (3.0 g 0.015 mol) was stirred in a mixture of water (60 ml), tetrahydrofuran (100 ml), o-methylbenzylamine (1.83 g, 0.015 mol) and sodium acetate (1.38 g, 0.017 mol) for a total of 16 hours. The reaction mixture was evaporated under reduced pressure and crystallized from ethanol to give 2-chloro-4-(2-methylbenzylamino)quinazoline (1.44 g, 30%) m.p. 215-217°C.

B. Preparation of 2-amino-4-(2-methylbenzylaminoquinazoline hydrochloride

Substituting 2-chloro-4-(2-methylbenzylamino)quinazoline (1.3 g, 0.004 mol) for 2-chloro-4-(2-methylphenylamino)-8-methoxyquinazoline and using corresponding molar proportions of the other reagents in the Example 1E gave after crystallisation from ethanolic hydrogen chloride/ether 2-amino-4-(2-methylbenzylamino)quinazoline hydrochloride (0.5 g, 42%), m.p. 258-260°C.

| | $C_{16}H_{16}N_4$ 1.0HCl 0.1$H_2O$ |
|---|---|
| Found | C 63.41, H 5.69, N 18.54, Cl 11.90 |
| Requires | C 63.51, H 5.73, N 18.51, Cl 11.71 |

Example 26

Preparation of 2-(2-methylphenylamino)-4-(N-methylphenylamino)quinazoline hydrochloride

A. Preparation of 2-chloro-4-(N-methylphenylamino)quinazoline

Substituting N-methylphenylamine (9.9 g, 0.092 mol) for 2-methyl-benzylamine and using corresponding molar proportions of the other reagents in Example 25A, gave after crystallisation from ethanol/water 2-chloro-4-(N-methylphenylamino)quinazoline hydrochloride (24.47 g, 75%), m.p. >300°C.

B. Preparation of 2-(2-methylphenylamino)-4-(N-methylphenylamino)quinazoline hydrochloride

Substituting 2-chloro-4-(N-methylphenylamino)quinazoline (2.0 g, 0.007 mol) for 2-chloro-8-methoxy-4-(2-methylphenylamino)quinazoline and using corresponding molar proportions of the other reagents in Example 12, gave after crystallisation from ethanolic hydrogen chloride 2-(2-methylphenylamino)-4-(N-methylphenylamino)quinazoline hydrochloride (1.36 g, 48%), m.p. 255-257°C.

| | $C_{22}H_2ON_4$ 1.0HCl |
|---|---|
| Found | C 69.95, H 5.58, N 14.89, Cl 9.38 |
| Requires | C 70.11, H 5.62, N 14.87, Cl 9.41 |

Example 27

Preparation of 2-phenylamino-4-(N-methylphenylamino)quinazoline hydrochloride

2-Chloro-4-(N-methylphenylamino)quinazoline (2.0 g, 0.007 mol) was dissolved in ethanol (20 ml) with aniline (1.37 g, 0.15 mol) and heated in a sealed vessel at 150°C for 5 hours. After cooling and removal of excess solvent in vacuo pressure the solid was treated with ethanolic hydrogen chloride to form the hydrochloride which was recrystallized from ethanol to give 2-phenylamino-4-(N-methylphenylamino)quinazoline hydrochloride (1.99 g, 74%), m.p. 265-267°C.

| | $C_{21}H_{18}N_4$ 1.0HCl |
|---|---|
| Found | C 69.28, H 5.17, N 15.35, Cl 9.78 |
| Requires | C 69.51, H 5.28, N 15.44, Cl 9.77 |

Example 28

Preparation of 2-amino-4-(N-methylphenylamino)quinazoline hydrochloride

Substituting N-methylaniline (1.95 g, 0.0183 mol) for O-toluidine and using corresponding molar proportions of the other reagents in Example 7C, gave after crystallisation from ethanolic hydrogen chloride 2-amino-4-(N-methylphenylamino)quinazoline hydrochloride (0.27 g, 13%), m.p. 190-192°C.

| | $C_{15}H_{14}N_4$ 0.3$H_2O$ 0.15HCl |
|---|---|
| Found | C 68.43, H 5.48, N 21.59, Cl 2.17 |
| Requires | C 68.81, H 5.70, N 21.40, Cl 2.04 |

Example 29

Preparation of 2-amino-4-(N-methylphenylamino)-8-methoxyquinazoline hydrochloride

Substituting 2-amino-4-chloro-8-methoxyquinazoline for 2-amino-4-chloroquinazoline and using corresponding molar proportions of the other reagents in Example 28, gave after crystallisation from ethanolic hydrogen chloride 2-amino-4-(N-methylphenylamino)-8-methoxyquinazoline hydrochloride (0.27 g, 10%), m.p. 282-284°C.

| | $C_{16}H_{16}N_4O$ HCl |
|---|---|
| Found | C 60.34, H 5.60, N 17.67, Cl 10.98 |
| Requires | C 60.66, H 5.38, N 17.69, Cl 11.19 |

Example 30

Preparation of 2-[(4-hydroxy-2-methylphenyl)amino]-4-(N-methylphenylamino) quinazoline hydrochloride

Substituting 4-hydroxy-2-methylaniline (1.8 g 0.0148 mol) for aniline and using corresponding molar proportions of the other reagents in Example 27, gave after crystallisation from ethanolic hydrogen chloride 2-[(4-hydroxy-2-methylphenyl)amino]quinazoline hydrochloride (0.72 g, 24%), m.p. 274-276°C.

| | $C_{22}H_{20}N_4O$ 1.0HCl |
|---|---|
| Found | C 66.91, H 5.33, N 14.06, Cl 8.97 |
| Requires | C 67.25, H 5.39, N 14.26, Cl 9.02 |

Example 31

Preparation of 2-(2-methylbenzylamino)-4-(N-methylphenylamino)quinazoline hydrochloride

Substituting 2-methylbenzylamine (1.79 g, 0.0148 mol) for aniline and using corresponding molar proportions of the other reagents in Example 27, gave after crystallisation from ethanolic hydrogen chloride 2-(2-methylbenzylamino)-4-(N-methylphenylamino)quinazoline hydrochloride (0.61 g, 21%), m.p. 226-228°C.

| | $C_{23}H_{22}N_4$ 1.0HCl |
|---|---|
| Found | C 70.44, H 5.90, N 14.36, Cl 9.08 |
| Requires | C70.67, H 5.93, N 14.33, Cl 9.07 |

Example 32

Preparation of 2-[(2-methyl-4-fluorophenyl)amino]-4-(N-methylphenylamino)quinazoline hydrochloride

Substituting 2-methyl-4-fluoroaniline (1.85 g, 0.0148 mol) for aniline and using corresponding molar proportions of the other reagents in Example 27, gave after crystallisation from ethanolic hydrogen chloride 2-[(2-methyl-4-fluorophenyl)amino]-4-(N-methylphenylamino)quinazoline hydrochloride (0.6 g, 15%), m.p. 243-245°C.

| | $C_{22}H_{19}N_4F$ 1.0HCl |
|---|---|
| Found | C 66.50, H 5.24, N 14.17, Cl 8.93 |
| Requires | C 66.92, H 5.11, N 14.19, Cl 8.98 |

Example 33

Preparation of 2-(2-methylphenylamino)-4-phenylaminoquinazoline hydrochloride

A. Preparation of 2-(2-methylphenylamino)-4-quinazolone

2-Methylthio-4-quinazolone (10.0 g, 0.052 mol) was fused with o-toluidine (8.35 g, 0.078 mol) at 160°C. After 4 hours, the solid was treated with ethanol, and filtered to give 2-methylphenylamino)-4-quinazolone (10.7 g, 82%) m.p. 278-280°C.

B. Preparation of 4-chloro-2-(2-methylphenylamino)quinazoline

2-(2-Methylphenylamino)-4-quinazolone (5.0 g, 0.0198 mol) was dissolved in phosphoryl chloride (20 ml, 0.216 mol) and N,N-dimethylaniline (3.5 ml, 0.025 mol) and refluxed for 3/4 hours. The reaction mixture was poured onto ice/N NaoH (100 ml) and the precipitate washed and dried to give 4-chloro-2-(2-methylphenylamino)quinazoline hydrochloride (5.79 g, 95%), used without purification.

C. Preparation of 2-(2-methylphenylamino)-4-phenylaminoquinazoline hydrochloride

4-Chloro-2-(2-methylphenylamino)quinazoline (1.5 g, 0.0048 mol) was dissolved in aniline (1.5 ml, 0.016 mol) and heated at 170°C for 1 hour. After cooling and evaporation of excess solvent the residue was crystallised from ethanolic hydrogen chloride to give 2-(2-methylphenylamino)-4-phenylaminoquinazoline hydrochloride (0.30 g, 17%) m.p. 239-240°C.

| | $C_{21}H_{18}N_4$ 1.0HCl 0.19$H_2O$ 0.02EtOH |
|---|---|
| Found | C 68.96, H 5.31, N 15.42, Cl 9.59 |
| Requires | C 68.71, H 5.37, N 15.22, Cl 9.63 |

Example 34

Preparation of 2-(2-methylphenylamino)-4-methylaminoquinazoline hydrochloride

2-(2-methylphenylamino)-4-chloroquinazoline (2 g, 0.0065 mol) and methylamine in ethanol (33%, 30 ml) were placed in a pressure vessel and heated for 4 hours at 140°C. After cooling, the reaction mixture was evaporated to dryness. The residue afforded crystals of 2-(2-methylphenylamino)-4-methylaminoquinazoline hydrochloride (0.29 g, 8.9%) from ethanolic hydrogen chloride, m.p. 275-279°C.

| | $C_{16}H_{16}N_4$ HCl |
|---|---|
| Found | C 63.70, H 5.56, N 18.58, Cl 11.76 |
| Requires | C 63.89, H 5.70, N 18.63, Cl 11.79 |

Example 35

Preparation of 2-(2-methylphenylamino)-4-propylaminoquinazoline hydrochloride

Substituting n-propylamine (1.77 g, 0.03 mol) for methylamine and using corresponding molar proportions of the other reagents in Example 34, gave after crystallisation 2-(2-methylphenylamino)-4-propylaminoquinazoline hydrochloride (0.67 g, 20.5%) from ethanolic hydrogen chloride, m.p. 215-217°C.

| | $C_{17}H_{18}N_4$ HCl |
|---|---|
| Found | C 65.12, H 6.20, N 17.83, Cl 11.20 |
| Requires | C 64.86, H 6.08, N 17.80, Cl 11.26 |

Example 36

Preparation of 2-(2-methylphenylamino)-4-(n-pentylamino)quinazoline hydrochloride

Substituting amylamine (1.13 g, 0.013 mol) for methylamine and using corresponding molar proportions of the other reagents in Example 34, gave after crystallisation 2-(2-methylphenylamino)-4-(n-pentylamino)quinazoline hydrochloride (0.24 g, 10.3%) from ethanolic hydrogen chloride/ether, m.p. 129-130°C.

| | $C_{20}H_{24}N_4$ HCl |
|---|---|
| Found | C 67.37, H 7.19, N 15.46, Cl 9.86 |
| Requires | C 67.31, H 7.06, N 15.70, Cl 9.93 |

Example 37

Preparation of 2-(2-methylphenylamino)-4-(2-methoxybenzylamino)quinazoline hydrochloride

Substituting 2-methoxybenzylamine (1.78 g, 0.0147 mol) for methylamine and using corresponding molar proportions of the other reagents in Example 34, gave after crystallisation 2-(2-methylphenylamino)-4-(2-methoxybenzylamino)quinazoline hydrochloride (1.52 g, 57.5%) from ethanolic hydrogen chloride, m.p. 218-220°C.

$C_{23}H_{22}N_4$ OHCl

Example 38

Preparation of 2-(2-methylphenylamino)-4-N-piperidinoquinazoline hydrochloride

Substituting piperidine (1.25 g, 0.0147 mol) for methylamine and using corresponding molar proportions of the other reagents in Example 34, gave after crystallisation 2-(2-methylphenylamino)-4-N-piperidinoquinazoline hydrochloride (0.25 g, 10%) from ethanolic hydrogen chloride/ether, m.p. 218-220°C.

| | $C_{20}H_{22}N_4$ HCl |
|---|---|
| Found | C 67.48, H 6.51, N 15.63, Cl 9.94 |
| Requires | C 67.69, H 6.53, N 15.79, Cl 9.99 |

Example 39

Preparation of 2-(2-methylphenylamino)-4-N-morpholinoquinazoline hydrochloride

Substituting morpholine (1.28 g, 0.0147 mol) for methylamine and using corresponding molar proportions of the other reagents in Example 34, gave after crystallisation 2-(2-methylphenylamino)-4-N-morpholinoquinazoline hydrochloride (0.26 g, 11.2%), from ethanolic hydrogen chloride/ether, m.p. 244-246°C.

| | $C_{19}H_{20}N_4O$ HCl |
|---|---|
| Found | C 64.04, H 6.03, N 15.80, Cl 10.04 |
| Requires | C 63.95, H 5.93, N 15.70, Cl 9.94 |

Example 40

Preparation of 2-(2-methylphenylamino)-4-dimethylaminoquinazoline hydrochloride

Substituting dimethylamine (30 ml) for methylamine and using corresponding molar proportions of the other reagents in Example 34, gave after crystallisation 2-(2-methylphenylamino)-4-dimethylaminoquinazoline hydrochloride (0.29 g, 11.8%), m.p. 277-282°C decomp.

| | $C_{17}H_{18}N_4$ HCl |
|---|---|
| Found | C 65.10, H 6.20, N 17.83, Cl 11.20 |
| Requires | C 64.86, H 6.08, N 17.80, Cl 11.26 |

Example 41

Preparation of 2-(2-methyl-4-fluorophenylamino)-4-(N-methylphenylamino)-8-methoxyquinazoline hydrochloride

2-Chloro-4-N-methylphenylamino-8-methoxyquinazoline (0.8 g, 0.0026 mol) and 4-fluoro-2-methylaniline (0.66 g, 0.0053 mol) were dissolved in ethanol (20 ml) placed in a pressure vessel and heated for 4 hours at 150°C. After cooling the reaction mixture was evaporated to drynesss. The residue afforded crystals of 2-(2-methyl-4-fluorophenylamino)-4-(N-methylphenylamino-8-methoxyquinazoline hydrochloride (0.25 g, 22%) from ethanolic hydrogen chloride, m.p. 218-220°C.

| | $C_{23}H_{21}N_4FO$ HCl |
|---|---|
| Found | C 64.87, H 5.19, N 13.05, Cl 8.16 |
| Requires | C 65.01, H 5.22, N 13.19, Cl 8.34 |

Example 42

Preparation of 2-[(4-methoxy-2-methylphenyl)amino]-4-(N-methylphenylamino)quinazoline hydrochloride

A. Preparation of 2-[(4-methoxy-2-methylphenyl)amino]quinazolin-4-one

Substituting 4-methoxy-2-methylaniline (5.48 g) for o-toluidine and using corresponding molar proportions of other reagents in Example 33A gave 2-[(4-methoxy-2-methylphenyl)amino]quinazolin-4-one (4.35 g, 77%) crystallised from methanol, m.p. 270-272°C.

B. Preparation of 4-chloro-2-[(4-methoxy-2-methylphenyl)amino]quinazoline

Substituting 2-[(4-methoxy-2-methylphenyl)amino]quinazolin-4-one (3.6 g) for 2-(2-methylphenylamino)quinazolin-4-one and using corresponding molar proportions of other reagents in Example 33B gave 4-chloro-2-[(4-methoxy-2-methylphenyl)amino]quinazoline (3.8 g) which was used without purification.

C. Preparation of 2-[(4-methoxy-2-methylphenyl)amino]-4-(N-methylphenylamino)quinazoline hydrochloride

Substituting 4-chloro-2-[(4-methoxy-2-methylphenyl)amino]quinazoline (3.8 g) for 4-chloro-2-[(2-methylphenyl)amino]quinazoline and N-methylaniline for aniline in Example 33C gave the title compound (2.0 g, 38%-2 steps) which was recrystallised from ethanol/diethyl ether as yellow needles, m.p. 248-250°C.

| | $C_{23}H_{22}N_4O.HCl.0.25\ H_2O$ |
|---|---|
| Found | C 67.01, H 5.72, N 13.52, Cl 8.33 |
| Requires | C 67.14, H 5.76, N 13.62, Cl 8.62 |

Example 43

Preparation of 4-(N-methylphenylamino)-2-(2-methylphenylamino)-6-methoxyquinazoline hydrochloride

A. 2,4-Dihydroxy-6-methoxyquinazoline

A mixture of 5-methoxy anthranilic acid (40 g, 0.24 M), acetic acid (18.0 g, 0.3 M) and 1 litre warm water (35°C) was stirred and allowed to cool. A solution of potassium cyanate (24.3 g, 0.3 M) in water (100 ml) was added over 15 minutes and then stirred for a further 30 minutes. Sodium hydroxide (320 g, 8.0 M) solid was added portionwise and the reaction was then heated at 90°C for 30 minutes and allowed to cool overnight. The solid was filtered off and dissolved in hot water (500 ml) and then acidified with dilute sulphuric acid to precipitate the product. The solid was filtered off and washed free of acid with water and then dried at 100°C/vacuum to yield the title compound (37.7 g) m.p. >290°C.

B. 2,4-Dichloro-6-methoxyquinazoline

2,4-Dihydroxy-6-methoxyquinazoline (37 g, 0.19 M), phosphoryl chloride (95 ml) and dimethylaniline (40 ml) were heated under reflux for 3 hours. After cooling the reaction mixture was poured onto ice and the precipitated solid was filtered off, washed with water and air dried. The product was used immediately in the next stage.

C. 2-Chloro-4-(N-methylphenylamino)-6-methoxyquinazoline

A mixture of 2,4-dichloro-6-methoxyquinazoline, (38.3 g. 0.19 M assuming 100% yield from previous step), N-methylaniline (19.26 g, 0.18 M), sodium acetate (16.4 g, 0.2 M) in tetrahydrofuran (2 litres) and water (1 litre) was stirred at room temperature for 8 days. Reaction was then concentrated to low volume (about 500 ml) and an oil separated out which solidified. The solid was filtered off and extracted with dichloromethane. Silica gel column chromatography followed by evaporation to dryness gave a residue which was washed with 40-60°C petroleum ether (to remove trace impurity) to yield title compound (31.25 g) m.p. 133-4°C.

D. Preparation of 4-(N-methylphenylamino)-2-(2-methylphenylamino)-6-methoxyquinazoline hydrochloride

A mixture of 2-chloro-4-(N-methylphenylamino)-6-methoxyquinazoline (5.4 g, 0.027 M), o-toluidine (4.28 g, 0.04 M) in ethanol (50 ml) was heated in a Berchof pressure vessel at 140°C for 5 hours (maximum pressure 40 psi). After cooling, ethanol/HCl was added and the solid which crystallised out was filtered off and recrystallised from isopropanol/ether to yield the title compound (4.23 g, m.p. 258-260°C).

Example 44

4-(N-Methylphenylamino)-2-(2-methyl-4-fluorophenylamino)-6-methoxyquinazoline

2-Chloro-4-(N-methylphenylamino)-6-methoxyquinazoline (5.4 g, 0.02 M) and 4-fluoro-2-methylaniline (4.96 g, 0.04 M) were dissolved in ethanol (50 ml) and heated at 140°C for 5 hours in a Berghof pressure reactor. The mixture was cooled and ethereal/HCl added. The precipitated solid was filtered off and recrystallised (isopropanol/ether followed by isopropanol) to yield the title compound (3.8 g) m.p. 248-250°C.

Example 45

Preparation of 2-(2-methylphenylamino)-4-(N-methyl-4-methoxyphenylamino)quinazoline hydrochloride

Substituting N-methyl-p-anisidine (3.18 g, 0.023 mol) for methylamine and using corresponding molar proportions of the other reagents in Example 34, gave after crystallisation 2-(2-methylphenylamino)-4-(N-methyl-4-methoxyphenylamino)quinazoline hydrochloride (2.45 g, 33%), from ethanolic hydrogen chloride, m.p. 224-226°C.

Example 46

Preparation of 2-(2-methylphenylamino)-4-(N-methyl-4-hydroxyphenylamino)quinazoline hydrochloride

2-(2-Methylphenylamino)-4-(N-methyl-4-methoxyphenylamino)quinazoline hydrochloride (1.6 g, 0.0043 mol) was stirred in dry dichloromethane (50 ml) at 0-5°C under nitrogen. To this solution was added boron tribromide (2.0 ml, 0.0216 mol) dropwise at 0-5°C over 10 minutes. The mixture was stirred for 3 hours at 0-5°C and then allowed to reach room temperature over 16 hours. After pouring onto ice, basifying and neutralising the aqueous phase was extracted with dichloromethane, the organic extracts which dried and evaporated to dryness. The residue afforded crystals of 2-(2-methylphenylamino)-4-(n-methyl-4-hydroxyphenylamino)quinazoline hydrochloride (0.4 g, 23.7%) from ethanolic hydrogen chloride m.p. 317-319°C.

Example 47

Preparation of 2-[(4-chloro-2-methylphenyl)amino]-4-(N-methylphenylamino)quinazoline hydrochloride.

A. Preparation of 2-[(4-chloro-2-methylphenyl)amino]-4-(N-methylphenylamino)quinazolone.

Substituting 2-methyl-4-chloroaniline (2.6 g) for o-toluidine and using corresponding molar proportions of the other reagents in Example 33A gave the title compound (2.73 g), m.p. >270°C (dec).

B. Preparation of 4-chloro-2-[(4-chloro-2-methylphenyl)amino]quinazoline.

Substituting 2-[(4-chloro-2-methylphenyl)amino]-4-quinazolone (2.5 g) for 2-[(2-methylphenyl)amino]-4-quinazolone and using corresponding molar proportions of the other reagents in Example 33B gave the title compound (1.0 g) which was used without purification.

C. Preparation of 2-[(4-chloro-2-methylphenyl)amino]-4-(N-methylphenylamino)quinazoline hydrochloride

Substituting 4-chloro-2-[(4-chloro-2-methylphenyl)amino]quinazoline (1.0 g) and N-methylaniline (1.0 g) for the reagents in Example 33C and using analogous conditions and work-up gave the title compound (0.17 g), m.p. 263-265°C, after two recrystallisations from ethanol/diethyl ether.

Example 48

Preparation of 2-(2-methylphenylamino)-4-(N-methylphenylamino)-8-fluoroquinazoline hydrochloride

In a procedure analogous to that of Example 41 2-chloro-4-(N-methylphenylamino)-8-fluoroquinazoline and o-toluidine are reacted together to give the title compound. The starting 2-chloro-4-(N-methylphenylamino)-8-fluoroquinazoline is prepared via procedures analogous to those of Example 1.

Example 49

Preparation of 2-(2-methyl-4-fluorophenylamino)-4-(N-methylphenylamino)-8-fluoroquinazoline

In a procedure analogous to that of Example 41 2-chloro-4-(N-methylphenylamino)-8-fluoroquinazoline and 2-methyl-4-fluoroaniline are reacted together to give the title compound.

Example 50

Preparation of 2-(2-methylphenylamino)-4-(N-ethylphenylamino)quinazoline hydrochloride

A. Preparation of 4-(N-ethylphenylamino)-2-chloroquinazoline.

Substituting N-ethylaniline for N-methylphenylamine and using corresponding molar proportions of other reagents in Example 26A, gives the title compound.

B. Preparation of 2-(2-methylphenylamino)-4-(N-ethylphenylamino)quinazoline hydrochloride.

Substituting 4-(N-ethylphenylamino)-2-chloroquinazoline for 4-(N-methylphenylamino)-2-chloroquinazoline and using molar proportions of the other reagents in Example 26B gives the title compound.

Biological Data.

(A) $H^+K^+$ATPase Activity.

The effects of a single high concentration (100 μM) of a compound of structure (I) on K-stimulated ATPase activity in lyophilised gastric vesicles was determined. Preferred compounds of structure (I) were also tested over a range of concentrations to determine $IC_{50}$ values.

(i) Preparation of lyophilised gastric vesicles (H/K-ATPase).

Lyophilised gastric vesicles were prepared from pig fundic mucosa after the method of Keeling et. al. (Biochem. Pharmacol., 34, 2967, 1985).

(ii) $K^+$-stimulated ATPase activity.

$K^+$-stimulated ATPase activity was determined at 37°C in the presence of the following : 10 mM Pipes/Tris buffer pH 7.0, 2 mM $MgSO_4$, 1 mM KCl, 2 mM $Na_2$ATP and 3-6 μg protein/ml lyophilised gastric vesicles. After incubation for 30 minutes, the inorganic phosphate hydrolysed from ATP was determined by the method of Yoda and Hokin (Biochem. Biophys. Res. Commun. 40, 880, 1970).

Compounds of structure (I) were dissolved in dimethylsulphoxide which up to the highest concentration used had no effect on $K^+$-stimulated ATPase activity.

The effect of the highest concentration of each compound of structure (I) on the recovery of a standard amount of inorganic phosphate was also determined.

(iii) Results.

The compounds of the examples had $IC_{50}$ values in the range of from 0.02 to 30 μM.

B. Rat : Lumen perfused stomach (pentagastrin stimulated gastric acid secretion).

Using a modification of the procedure described by Ghosh and Schild (Br. J. Pharmacology, 13, 54, 1958), the compounds of the following Examples were found on i.v. administration at a concentration of 10μmole/kg to cause an inhibition of pentagastrin stimulated gastric acid secretion as indicated in the following Table.

| Compound | Rat G.S. % inhibition @ 10μmole/kg |
|---|---|
| 1 | 60 |
| 2 | 25 |
| 3 | 16 |
| 4 | 37 |
| 5 | 37 |
| 6 | 48 |
| 7 | 20 |
| 8 | 17 |
| 11 | 28 |
| 13 | 20 |
| 15 | 31 |
| 16 | 31 |
| 18 | 12 |
| 20 | 72 |
| 21 | 37 |
| 22 | 27 |
| 24 | 97 |
| 25 | 24 |
| 26 | 79 |
| 27 | 71 |
| 29 | 13 |
| 30 | 23 |
| 31 | 8 |
| 32 | 96 |

Example A

A tablet for oral administration is prepared by combining

| | Mg/Tablet |
|---|---|
| Compound of structure (I) | . 100 |
| lactose | 153 |
| Starch | 33 |
| crospovidone | 12 |
| microcrystalline cellulose | 30 |
| magnesium stearate | 2 |
| | 330 mg |

into a 9 mm tablet.

Example B

An injection for parenteral administration was prepared from the following

| | %w:w |
|---|---|
| Compound of Example 20 | 0,50% (w:v) |
| 1M citric acid | 30% (v:v) |
| sodium hydroxide (qs) | to pH 3.2 |
| water for injection EP | to 100 ml |

The compound of Example 20 was dissolved in the citric acid and the pH slowly adjusted to pH 3.2 with the sodium hydroxide solution. The solution was then made up to 100 ml with water, sterilised by filtration and sealed into appropriately sized ampoules and vials.

## Claims

**Claims for the Contracting States : AT, BE, CH, DE, FR, GB, IT, LI, LU, NL, SE.**

1. A compound of structure (I)

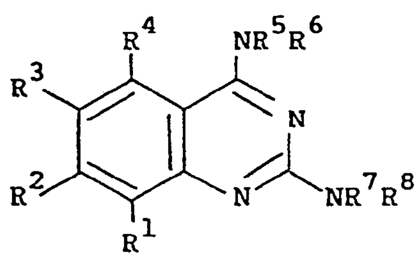

(I)

in which
$R^1$ to $R^4$ are the same or different and are each hydrogen, $C_{1-4}$alkyl, $C_{1-4}$alkoxy, phenyl, $C_{1-4}$alkylthio, $C_{1-4}$alkanoyl, amino, $C_{1-6}$alkylamino, di$C_{1-4}$alkylamino, halogen or trifluoromethyl provided that at least two of $R^1$ to

$R^4$ are hydrogen.
$R^5$ and $R^6$ are the same, or different and are each hydrogen, $C_{1-4}$alkyl, $-(CH_2)_nAr$ in which n is 0 to 4 and Ar is a phenyl group optionally substituted by 1-3 substuents selected from $C_{1-4}$alkyl, $C_{1-4}$alkoxy, $C_{1-4}$alkylthio, halogen, cyano, amino, hydroxy, carbamoyl, carboxy, $C_{1-4}$alkanoyl and trifluoromethyl or $R^5$ and $R^6$ together with the nitrogen atom to which they are attached form a saturated or unsaturated carbocyclic ring ; and :
$R^7$ and $R^8$ are the same or different and are each hydrogen, $C_{1-4}$alkyl; $(CH_2)_nAr^1$in which n is 0 to 4 and $Ar^1$ is a phenyl group optionally substituted by 1 to 3 substituents as described for Ar above, or $R^7$ and $R^8$ together with the nitrogen atom to which they are attached form a saturated or unsaturated carbocyclic ring ;
or a pharmaceutically acceptable salt thereof, provided that
° $R^5$, $R^6$, $R^7$ and $R^8$ are not all, at the same time, hydrogen ;
° at least one of $R^5$, $R^6$, $R^7$ and $R^8$ is a $(CH_2)_nAr$ or $(CH_2)_nAr^1$ group as appropriate ; and
° when $R^1$ and $R^4$ are both hydrogen, and one of $R^2$ and $R^3$ is $C_{1-4}$alkoxy, the other is not hydrogen or $C_{1-4}$alkoxy.

2. A compound according to claim 1 in which $R^2$ to $R^4$ are hydrogen and $R^1$ is hydrogen or $C_{1-4}$ alkoxy.

3. A compound according to claim 2 in which one of $R^5$ and $R^6$ is $(CH_2)_nAr$ in which n is 0 to 4 and Ar is a phenyl group optionally substituted as defined in claim 1 and the other is $C_{1-4}$alkyl.

4. A compound according to claim 3 in which n is 0.

5. A compound according to claim 4 in which one of $R^7$ and $R^8$ is hydrogen and the other is $-(CH_2)_nAr^1$ in which n is 0 to 4 and $Ar^1$ is phenyl ring optionally substituted as defined in claim 1.

6. A compound according to claim 1 which is
2-amino-8-methoxy-4-(2-methylphenylamino)quinazoline
2.4-Bis-(N-methylphenylamino)quinazoline
4-(N-methylphenylamino)-2-(2-methylphenylamino)-8-methoxyquinazoline
2-(2-methylphenylamino)-4-(N-methylphenylamino)quinazoline
2-phenylamino-4-(N-methylphenylamino)quinazoline
2-[(2-methyl-4-fluorophenyl)amino]-4-(N-methylphenylamino)quinazoline
2-(2-methylphenylamino)-4-phenylaminoquinazoline
or a pharmaceutically acceptable salt thereof,

7. A pharmaceutical composition comprising a compound according to any one of claims 1 to 6 and a pharmaceutical carrier.

8. A compound according to any one of claims 1 to 6 for use as a therapeutic agent.

9. A process for the preparation of a compound according to claim 1 which comprises :
(a) reaction of a compound of structure (II)

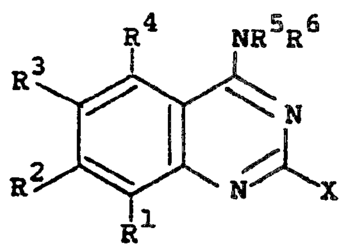

(II)

in which $R^1$ to $R^6$ are as described for structure (I) except that where necessary they are in protected form, and X is a group displaceable by an amine, with an amine of structure $R^7R^8NH$ in which $R^7$ and $R^8$ are as described for structure (I) ; or
(b) reaction of a compound of structure (III)

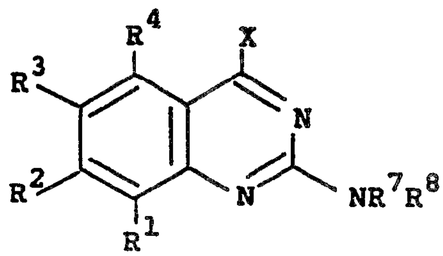

(III)

in which $R^1$ to $R^4$ and $R^7$ and $R^8$ are as described for structure (I) and X is a group displaceable by an amine, with an amine of structure $R^5R^6NH$ in which $R^5$ and $R^6$ are as described for structure (I) ; and optionally thereafter,

° removing any protecting groups ;

° forming a pharmaceutically acceptable salt.

10. A compound of structure (II)

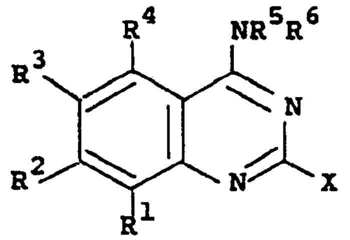

(II)

in which $R^1$ to $R^6$ are as described for structure (I) in claim 1 and and X is a group displaceable by an amine.

11. A compound of structure (III)

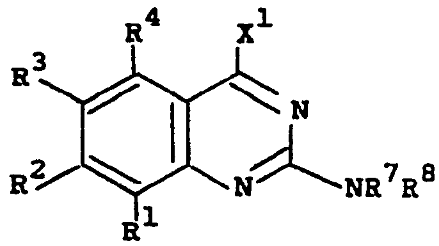

(III)

in which $R^1$ to $R^4$ and $R^7$ and $R^8$ are as described for structure (I) in claim 1 and X is a group displaceable by an amine.

12. The use of a compound of structure (IA)

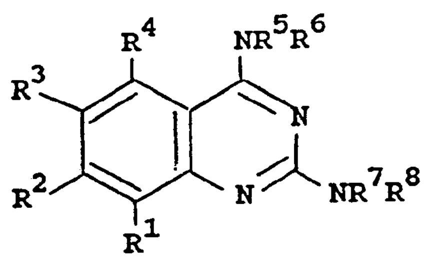

(IA)

in which

$R^1$ to $R^4$ are the same or different and are each hydrogen, $C_{1-4}$alkyl, $C_{1-4}$alkoxy, phenyl, $C_{1-4}$alkylthio, $C_{1-4}$alkanoyl, amino, $C_{1-6}$alkylamino, di$C_{1-4}$alkylamino, halogen or trifluoromethyl provided that at least two of $R^1$ to $R^4$ are hydrogen.

$R^5$ and $R^6$ are the same, or different and are each hydrogen, $C_{1-4}$alkyl, $-(CH_2)_nAr$ in which n is 0 to 4 and Ar is an optionally substituted phenyl group or $R^5$ and $R^6$ together with the nitrogen atom to which they are attached form a saturated'or unsaturated carbocyclic ring ; and ;

$R^7$ and $R^8$ are the same or different and are each hydrogen, $C_{1-4}$alkyl, $(CH_2)_nAr^1$ in which n is 0 to 4 and $Ar^1$ is an optionally substituted phenyl group, or $R^7$ and $R^8$ together with the nitrogen atom to which they are attached form a saturated or unsaturated carbocyclic ring ;

or a pharmaceutically acceptable salt thereof for the manufacture of a medicament for the treatment of gastrointestinal diseases.

**Claims for the Contracting State : ES.**

1. A process for the preparation of a compound of sctucture (I)

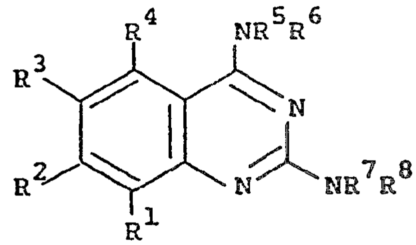

(I)

in which
$R^1$ to $R^4$ are the same or different and are each hydrogen, $C_{1-4}$alkyl, $C_{1-4}$alkoxy, phenyl, $C_{1-4}$alkylthio, $C_{1-4}$alkanoyl, amino, $C_{1-6}$alkylamino, di$C_{1-4}$alkylamino, halogen or trifluoromethyl provided that at least two of $R^1$ to $R^4$ are hydrogen.
$R^5$ and $R^6$ are the same, or different and are each hydrogen. $C_{1-4}$alkyl, $-(CH_2)_nAr$ in which n is 0 to 4 and Ar is a phenyl group optionally substituted by 1-3 substitituents selected from $C_{1-4}$alkyl, $C_{1-4}$alkoxy, $C_{1-4}$alkylthio, halogen, cyano, amino, hydroxy, carbamoyl, carboxy,, $C_{1-4}$alkanoyl and trifluoromethyl, or $R^5$ and $R^6$ together with the nitrogen atom to which they are attached form a saturated or unsaturated carbocyclic ring ; and :
$R^7$ and $R^8$ are the same or different and are each hydrogen, $C_{1-4}$ alkyl ; $(CH_2)_nAr^1$ in which n is 0 to 4 and $Ar^1$ is a phenyl group optionally substituted by 1 to 3 substituents as described for Ar above, or $R^7$ and $R^8$ together with the nitrogen atom to which they are attached form a saturated or unsaturated carbocyclic ring ;
or a pharmaceutically acceptable salt thereof provided that
* ° $R^5$, $R^6$, $R^7$ and $R^8$ are not all, at the same time, hydrogen ;
° at least one of $R^5$, $R^6$, $R^7$ and $R^8$ is a $(CH_2)_nAr$ or $(CH_2)_nAr^1$ group as appropriate ; and
° when $R^1$ and $R^4$ are both hydrogen, and one of $R^2$ and $R^3$ is $C_{1-4}$alkoxy, the other is not hydrogen or $C_{1-4}$alkoxy,
which comprises,
(a) reaction of a compound of structure (II)

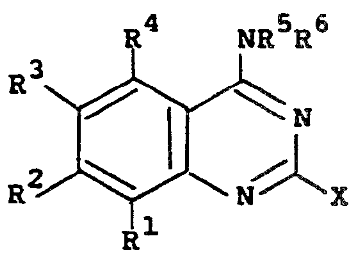

(II)

in which $R^1$ to $R^6$ are as described for structure (I) except that where necessary they are in protected form, and X is a group displaceable by an amine, with an amine of structure $R^7R^8NH$ in which $R^7$ and $R^8$ are as described for structure (I) ; or
(b) reaction of a compound of structure (III)

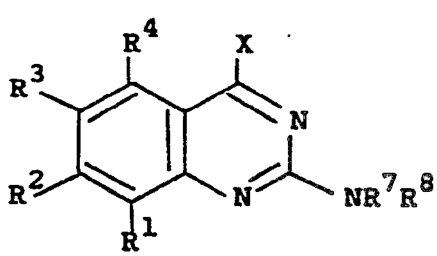

(III)

in which $R^1$ to $R^4$ and $R^7$ and $R^8$ are as described for structure (I) and X is a group displaceable by an amine,

with an amine of structure $R^5R^6NH$ in which $R^5$ and $R^6$ are as described for structure (I) ; and optionally thereafter,

° removing any protecting groups ;

° forming a pharmaceutically acceptable salt.

2. A process as claimed in claim 1(a) when carried out in the absence of solvent in a sealed receptacle at elevated temperature.

3. A process as claimed in claim 1(b) when carried out in an inert solvent at elevated temperature.

4. A process as claimed in claim 3 in which the solvent is a $C_{1-4}$alkanol.

5. A process as claimed in any one of claims 1 to 4, in which in the compound of structure (I). $R^2$ to $R^4$ are hydrogen and $R^1$ is hydrogen or $C_{1-4}$alkoxy.

6. A process as claimed in claim 5 in which in the compound of structure (I) one of $R^5$ and $R^6$ is $(CH_2)_nAr$ in which n is 0 to 4 and Ar is an optionally substituted phenyl ring as defined in claim 1 and the other is $C_{1-4}$alkyl.

7. A process as claimed in claim 6 in which in the compound of structure (I) n is 0.

8. A process as claimed in claim 7 in which in the compound of structure (I) one of $R^7$ and $R^8$ is hydrogen and the other is $-(CH_2)_nAr^1$ in which n is 0 to 4 and $Ar^1$ is an optionally substituted phenyl ring as defined in claim 1.

9. A process as claimed in any one of claims 1-8 in which the compound of structure (I) is
2-amino-8-methoxy-4-(2-methylphenylamino)quinazoline
2,4-Bis-(N-methylphenylamino)quinazoline
4-(N-methylphenylamino)-2-(2-methylphenylamino)-8-methoxyquinazoline
2-(2-methylphenylamino)-4-(N-methylphenylamino)quinazoline
2-phenylamino-4-(N-methylphenylamino)quinazoline
2-[(2-methyl-4-fluorophenyl)amino]-4-(N-methylphenylamino)quinazoline
2-(2-methylphenylamino)-4-phenylaminoquinazoline

10. A process for the preparation of a pharmaceutical composition which comprises bringing into association a compound of structure (I) or a pharmaceutically acceptable salt thereof as described in claim 1 and a pharmaceutical carrier.

11. A process according to claim 10, in which the compound of structure (I) is
2-amino-8-methoxy-4-(2-methylphenylamino)quinazoline
2,4-Bis-(N-methylphenylamino)quinazoline
4-(N-methylphenylamino)-2-(2-methylphenylamino)-8-methoxyquinazoline
2-(2-methylphenylamino)-4-(N-methylphenylamino)quinazoline
2-phenylamino-4-(N-methylphenylamino)quinazoline
2-[(2-methyl-4-fluorophenyl)amino]-4-(N-methylphenylamino)quinazoline
2-(2-methylphenylamino)-4-phenylaminoquinazoline

12. The use of a compound of structure (IA)

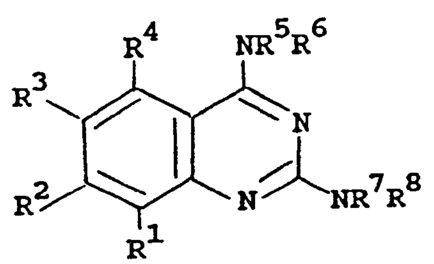

(IA)

in which

$R^1$ to $R^4$ are the same or different and are each hydrogen, $C_{1-4}$alkyl, $C_{1-4}$alkoxy, phenyl, $C_{1-4}$alkylthio, $C_{1-4}$alkanoyl, amino, $C_{1-6}$alkylamino, di$C_{1-4}$alkylamino, halogen or trifluoromethyl provided that at least two of $R^1$ to $R^4$ are hydrogen.

$R^5$ and $R^6$ are the same, or different and are each hydrogen, $C_{1-4}$alkyl, $-(CH_2)_nAr$ in which n is 0 to 4 and Ar is an optionally substituted phenyl group or $R^5$ and $R^6$ together with the nitrogen atom to which they are attached form a saturated or unsaturated carbocyclic ring ; and ;

$R^7$ and $R^8$ are the same or different and are each hydrogen, $C_{1-4}$alkyl, $(CH_2)_nAr^1$ in which n is 0 to 4 and $Ar^1$ is an optionally substituted phenyl group, or $R^7$ and $R^8$ together with the nitrogen atom to which they are attached form a saturated or unsaturated carbocyclic ring ;

or a pharmaceutically acceptable salt thereof for the manufacture of a medicament for the treatment of gas-

trointestinal diseases.

**Claims for the Contracting State : GR.**

1. A process for the preparation of a compound of structure (I)

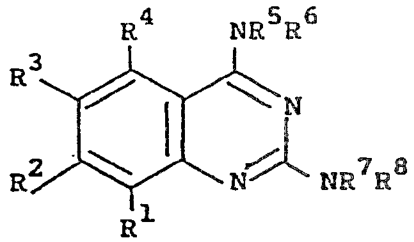

(I)

in which

$R^1$ to $R^4$ are the same or different and are each hydrogen, $C_{1-4}$alkyl, $C_{1-4}$alkoxy, phenyl, $C_{1-4}$alkylthio, $C_{1-4}$alkanoyl, amino, $C_{1-4}$alkylamino, di$C_{1-4}$alkylamino, halogen or trifluoromethyl provided that at least two of $R^1$ to $R^4$ are hydrogen.

$R^5$ and $R^6$ are the same, or different and are each hydrogen, $C_{1-4}$alkyl, $-(CH_2)_n$Ar in which n is 0 to 4 and Ar is a phenyl group optionally substituted by 1 to 3 substuents selected from $C_{1-4}$alkyl, $C_{1-4}$alkoxy, $C_{1-4}$alkylthio, halogen, cyano, amino, hydroxy, carbamoyl, carboxy, $C_{1-4}$alkanoyl and trifluoromethyl or $R^5$ and $R^6$ together with the nitrogen atom to which they are attached form a saturated or unsaturated carbocyclic ring ; and ;

$R^7$and $R^8$ are the same or different and are each hydrogen, $C_{1-4}$alkyl, $(CH_2)_n$$Ar^1$ in which n is 0 to 4 and $Ar^1$ is a phenyl group optionally substituted by 1 to 3 substituents as described for Ar above, or $R^7$and $R^8$ together with the nitrogen atom to which they are attached form a saturated or unsaturated carbocyclic ring ;

or a pharmaceutically acceptable salt thereof provided that

* ° $R^5$, $R^6$, $R^7$ and $R^8$ are not all, at the same time, hydrogen ;

° at least one of $R^5$, $R^6$, $R^7$ and $R^8$ is a $(CH_2)_n$Ar or $(CH_2)_n$$Ar^1$ group as appropriate ; and

° when $R^1$ and $R^4$ are both hydrogen, and one of $R^2$ and $R^3$ is $C_{1-4}$alkoxy, the other is not hydrogen or $C_{1-4}$alkoxy,

which comprises,

(a) reaction of a compound of structure (II)

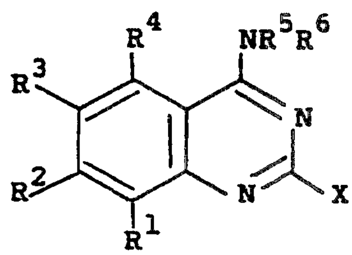

(II)

in which $R^1$ to $R^6$ are as described for structure (I) except that where necessary they are in protected form, and X is a group displaceable by an amine, with an amine of structure $R^7R^8NH$ in which $R^7$ and $R^8$ are as described for structure (I) ; or

(b) reaction of a compound of structure (III)

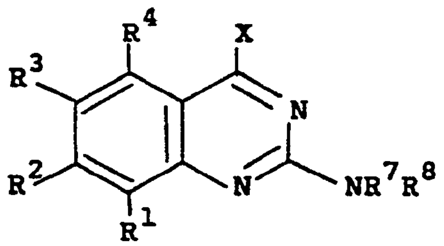

(III)

in which $R^1$ to $R^4$ and $R^7$ and $R^8$ are as described for structure (I) and X is a group displaceable by an amine, with an amine of structure $R^5R^6NH$ in which $R^5$ and $R^6$ are as described for structure (I) ; and optionally thereafter,

° removing any protecting groups ;

° forming a pharmaceutically acceptable salt.

2. A process as claimed in claim 1(a) when carried out in the absence of solvent in a sealed receptacle at elevated temperature.

3. A process as claimed in claim 1(b) when carried out in an inert solvent at elevated temperature.

4. A process as claimed in claim 3 in which the solvent is a $C_{1-4}$alkanol.

5. A process as claimed in any one of claims 1 to 4, in which in the compound of structure (I). $R^2$ to $R^4$ are hydrogen and $R^1$ is hydrogen or $C_{1-4}$ alkoxy.

6. A process as claimed in claim 5 in which in the compound of structure (I) one of $R^5$ and $R^6$ is $(CH_2)_nAr$ in which n is 0 to 4 and Ar is an optionally substituted phenyl ring as defined in claim 1 and the other is $C_{1-4}$alkyl.

7. A process as claimed in claim 6 in which in the compound of structure (I) n is 0.

8. A process as claimed in claim 7 in which in the compound of structure (I) one of $R^7$ and $R^8$ is hydrogen and the other is $-(CH_2)_nAr^1$ in which n is 0 to 4 and $Ar^1$ is an optionally substituted phenyl ring as defined in claim 1.

9. A process as claimed in any one of claims 1-8 in which the compound of structure (I) is
2-amino-8-methoxy-4-(2-methylphenylamino)quinazoline
2,4-Bis-(N-methylphenylamino)quinazoline
4-(N-methylphenylamino)-2-(2-methylphenylamino)-8-methoxyquinazoline
2-(2-methylphenylamino)-4-(N-methylphenylamino)quinazoline
2-phenylamino-4-(N-methylphenylamino)quinazoline
2-[(2-methyl-4-fluorophenyl)amino]-4-(N-methylphenylamino)quinazoline
2-(2-methylphenylamino)-4-phenylaminoquinazoline

10. A process for the preparation of a pharmaceutical composition which comprises bringing into association a compound of structure (I) or a pharmaceutically acceptable salt thereof as described in claim 1 and a pharmaceutical carrier.

11. A process according to claim 10, in which the compound of structure (I) is
2-amino-8-methoxy-4-(2-methylphenylamino)quinazoline
2.4-Bis-(N-methylphenylamino)quinazoline
4-(N-methylphenylamino)-2-(2-methylphenylamino)-8-methoxyquinazoline
2-(2-methylphenylamino)-4-(N-methylphenylamino)quinazoline
2-phenylamino-4-(N-methylphenylamino)quinazoline
2-[(2-methyl-4-fluorophenyl)amino]-4-(N-methylphenylamino)quinazoline
2-(2-methylphenylamino)-4-phenylaminoquinazoline

12. A compound of structure (IIA)

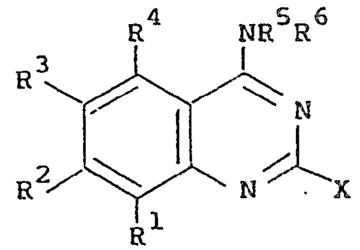

(IIA)

in which $R^1$ to $R^4$ are as described for structure (I) in claim 1, X is a group displaceable by an amine, $R^5$ is hydrogen or $C_{1-4}$alkyl and $R^6$ is $(CH_2)_nAr$ in which n and Ar are as described for structure (I).

13. A compound of structure (IIIA)

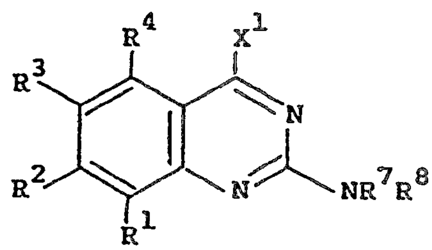

(IIIA)

in which $R^1$ to $R^4$ are as described for structure (I) in claim 1, X is a group displaceable by an amine, $R^7$ is hydrogen or $C_{1-4}$alkyl and $R^8$ is $(CH_2)_nAr^1$ in which n and $Ar^1$ are as described for structure (I).

14. The use of a compound of structure (IA)

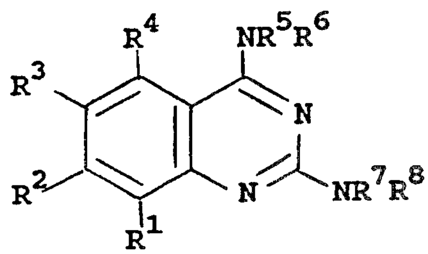

(IA)

in which

$R^1$ to $R^4$ are the same or different and are each hydrogen, $C_{1-4}$alkyl, $C_{1-4}$alkoxy, phenyl, $C_{1-4}$alkylthio, $C_{1-4}$alkanoyl, amino, $C_{1-6}$alkylamino, di$C_{1-4}$alkylamino, halogen or trifluoromethyl provided that at least two of $R^1$ to $R^4$ are hydrogen.

$R^5$ and $R^6$ are the same, or different and are each hydrogen, $C_{1-4}$alkyl, $-(CH_2)_nAr$ in which n is 0 to 4 and Ar is an optionally substituted phenyl group or $R^5$ and $R^6$ together with the nitrogen atom to which they are attached form a saturated or unsaturated carbocyclic ring ; and ;

$R^7$ and $R^8$ are the same or different and are each hydrogen, $C_{1-4}$alkyl, $(CH_2)_nAr^1$ in which n is 0 to 4 and $Ar^1$ is an optionally substituted phenyl group, or $R^7$ and $R^8$ together with the nitrogen atom to which they are attached form a saturated or unsaturated carbocyclic ring ;

or a pharmaceutically acceptable salt thereof for the manufacture of a medicament for the treatment of gastrointestinal diseases.

## Ansprüche

**Patentansprüche für die Vertragsstaaten : AT BE CH DE FR GB IT LI LU NL SE**

1. Verbindung der Struktur (I)

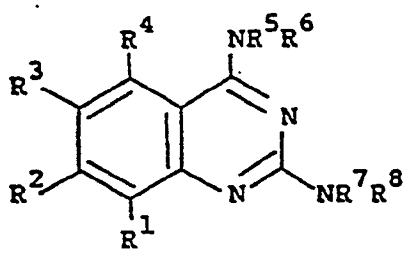

(I)

in der
$R^1$ bis $R^4$ gleich oder verschieden sind und jeweils Wasserstoffatome, $C_{1-4}$-Alkyl- oder $C_{1-4}$-Alkoxyreste, Phenylgruppen, $C_{1-4}$-Alkylthio- oder $C_{1-4}$-Alkanoylreste, Aminogruppen, $C_{1-6}$-Alkylamino- oder Di-$C_{1-4}$-alkylaminoreste, Halogenatome oder Trifluormethylgruppen bedeuten, mit der Maßgabe, daß mindestens zwei der Reste $R^1$ bis $R^4$ Wasserstoffatome sind ;
$R^5$ und $R^6$ gleich oder verschieden sind und jeweils Wasserstoffatome, $C_{1-4}$-Alkylreste oder die Gruppe $-(CH_2)_nAr$ darstellen, in der n den Wert 0 bis 4 annimmt und Ar eine gegebenenfalls mit 1 bis 3 Substituenten, ausgewählt aus $C_{1-4}$-Alkyl-, $C_{1-4}$-Alkoxy- oder $C_{1-4}$-Alkylthioreste, Halogenatome, Cyano-, Amino-, Hydroxy-, Carbamoyl- oder Carboxylgruppen, $C_{1-4}$-Alkanoylreste oder Trifluormethylgruppen, substituierte Phenylgruppe bedeutet oder $R^5$ und $R^6$ zusammen mit dem Stickstoffatom, an dem sie gebunden sind, einen gesättigten oder ungesättigten carbocyclischen Ring ergeben ;
und $R^7$ und $R^8$ gleich oder verschieden sind und jeweils Wasserstoffatome, $C_{1-4}$-Alkylreste oder die Gruppe $-(CH_2)_nAr^1$ darstellen, in der n den Wert 0 bis 4 annimmt und $Ar^1$ einen gegebenenfalls mit 1 bis 3 wie vorstehend für Ar beschriebenen Substituenten substituierte Phenylgruppe bedeutet oder $R^7$ und $R^8$ zusammen mit dem Stickstoffatom, an dem sie gebunden sind, einen gesättigten oder ungesättigten carbocyclischen Ring ergeben ;
oder ein pharmazeutisch verträgliches Salz davon, mit der Maßgabe, daß
$R^5$, $R^6$, $R^7$ und $R^8$ nicht alle gleichzeitig Wasserstoffatome sind ;
mindestens einer der Reste $R^5$, $R^6$, $R^7$ und $R^8$ je nach Eignung eine der Gruppen $-(CH_2)_nAr$ oder $-(CH_2)_nAr^1$ bedeutet ; und
falls $R^1$ und $R^4$ beide Wasserstoffatome bedeuten und einer der Reste $R^2$ und $R^3$ ein $C_{1-4}$-Alkoxyrest ist, der andere kein Wasserstoffatom oder $C_{1-4}$-Alkoxyrest ist.

2. Verbindung nach Anspruch 1, in der $R^2$ bis $R^4$ Wasserstoffatome sind und $R^1$ ein Wasserstoffatom oder ein $C_{1-4}$-Alkoxyrest ist.

3. Verbindung nach Anspruch 2, in der einer der Reste $R^5$ und $R^6$ die Gruppe $-(CH_2)_nAr$ bedeutet, in der n den Wert 0 bis 4 annimmt, und Ar eine gegebenenfalls gemäß Anspruch 1 substituierte Phenylgruppe bedeutet, und der andere ein $C_{1-4}$-Alkylrest ist.

4. Verbindung nach Anspruch 3, in der n den Wert 0 annimmt.

5. Verbindung nach Anspruch 4, in der einer der Reste $R^7$ und $R^8$ ein Wasserstoffatom bedeutet und der andere die Gruppe $-(CH_2)_nAr^1$ darstellt, in der n den Wert 0 bis 4 annimmt und $Ar^1$ eine gegebenenfalls gemäß Anspruch 1 substituierte Phenylgruppe bedeutet.

6. Verbindung nach Anspruch 1, nämlich
2-Amino-8-methoxy-4-(2-methylphenylamino)-chinazolin,
2,4-Bis-(N-methylphenylamino)-chinazolin,
4-(N-Methylphenylamino)-2-(2-methylphenylamino)-8-methoxychinazolin,
2-(2-Methylphenylamino)-4-(N-methylphenylamino)-chinazolin,
2-Phenylamino-4-(N-methylphenylamino)-chinazolin,
2-[(2-Methyl-4-fluorphenyl)-amino]-4-(N-methylphenylamino)-chinazolin oder
2-(2-Methylphenylamino)-4-phenylaminochinazolin,
oder ein pharmazeutisch verträgliches Salz davon.

7. Arzneimittel, umfassend eine Verbindung nach einem der Ansprüche 1 bis 6 und einen pharmazeutischen Träger.

8. Verbindung nach einem der Ansprüche 1 bis 6 zur Verwendung als therapeutisches Mittel.

9. Verfahren zur Herstellung einer Verbindung nach Anspruch 1, umfassend :

(a) Umsetzung einer Verbindung der Struktur (II)

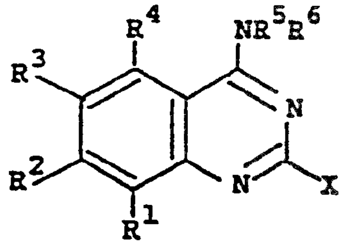

(II)

in der $R^1$ bis $R^6$ wie für Struktur (I) beschrieben sind, ausgenommen daß sie falls erforderlich in geschützter Form vorliegen, und X eine durch ein Amin ersetzbare Gruppe bedeutet, mit einem Amin der Struktur $R^7R^8NH$, in der $R^7$ und $R^8$ wie für Struktur (I) beschrieben sind ; oder

(b) Umsetzung einer Verbindung der Struktur (III)

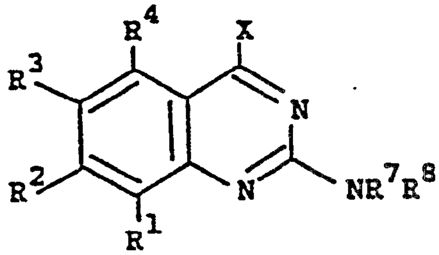

(III)

in der $R^1$ bis $R^4$ und $R^7$ und $R^8$ wie für Struktur (I) beschrieben sind und X eine durch ein Amin ersetzbare Gruppe bedeutet, mit einem Amin der Struktur $R^5R^6NH$, in der $R^5$ und $R^6$ wie für die Struktur (I) beschrieben sind ; und gegebenenfalls hiernach,

Entfernung jeglicher Schutzgruppen ;

Herstellen eines pharmazeutisch verträglichen Salzes.

10. Verbindung der Struktur (II)

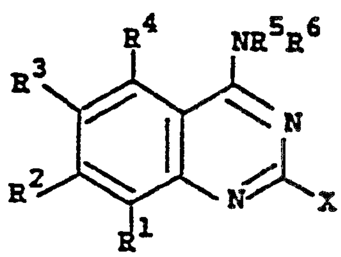

(II)

in der $R^1$ bis $R^6$ wie für Struktur (I) in Anspruch 1 beschrieben sind und X eine durch ein Amin ersetzbare Gruppe bedeutet.

11. Verbindung der Struktur (III)

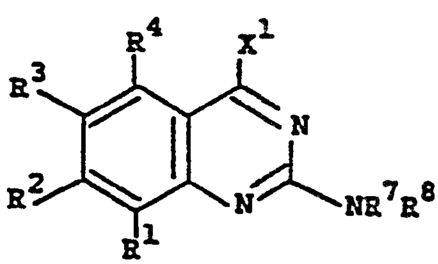

(III)

in der $R^1$ bis $R^4$ und $R^7$ und $R^8$ wie für Struktur (I) in Anspruch 1 beschrieben sind und X eine durch ein Amin ersetzbare Gruppe bedeutet.

12. Verwendung einer Verbindung der Struktur (IA)

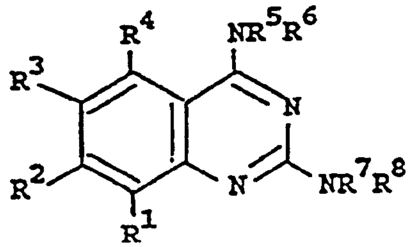

(IA)

in der
$R^1$ bis $R^4$ gleich oder verschieden sind und jeweils Wasserstoffatome, $C_{1-4}$-Alkyl- oder $C_{1-4}$-Alkoxyreste, Phenylgruppen, $C_{1-4}$-Alkylthio- oder $C_{1-4}$-Alkanoylreste, Aminogruppen, $C_{1-6}$-Alkylamino- oder Di-$C_{1-4}$-alkylaminogruppen, Halogenatome oder Trifluormethylgruppen bedeuten, mit der Maßgabe, daß mindestens zwei der Reste $R^1$ bis $R^4$ Wasserstoffatome sind ;
$R^5$ und $R^6$ gleich oder verschieden sind und jeweils Wasserstoffatome, $C_{1-4}$-Alkylreste, oder die Gruppe $-(CH_2)_nAr$ darstellen, in der n die Zahl 0 bis 4 annimmt und Ar eine gegebenenfalls substituierte Phenylgruppe bedeutet oder $R^5$ und $R^6$ zusammen mit dem Stickstoffatom, an dem sie gebunden sind, einen gesättigten oder ungesättigten carbocyclischen Ring ergeben ; und ;
$R^7$ und $R^8$ gleich oder verschieden sind und jeweils Wasserstoffatome, $C_{1-4}$-Alkylreste oder die Gruppe $-(CH_2)_nAr^1$ darstellen, in der n die Zahl von 0 bis 4 annimmt und $AR^1$ eine gegebenenfalls substituierte Phenylgruppe bedeutet, oder $R^7$ und $R^8$ zusammen mit dem Stickstoffatom, an dem sie gebunden sind, einen gesättigten oder ungesättigten carbocyclischen Ring ergeben ;
oder eines pharmazeutisch verträglichen Salzes davon zur Herstellung eines Arzneimittels zur Behandlung gastrointestinaler Erkrankungen.

**Patentansprüche für den Vertragsstaat : ES**

1. Verfahren zur Herstellung einer Verbindung der Struktur (I)

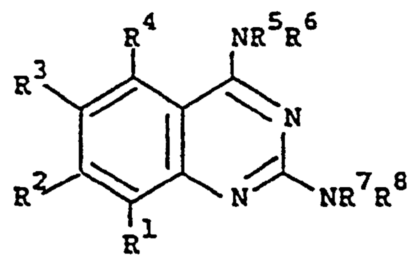

(I)

in der
$R^1$ bis $R^4$ gleich oder verchieden sind und jeweils Wasserstoffatome, $C_{1-4}$-Alkyl- oder $C_{1-4}$-Alkoxyreste, Phenylgruppen, $C_{1-4}$-Alkylthio- oder $C_{1-4}$-Alkanoylreste, Aminogruppen, $C_{1-6}$-Alkylamino- oder Di-$C_{1-4}$-alkylaminoreste, Halogenatome oder Trifluormethylgruppen bedeuten, mit der Maßgabe, daß mindestens zwei der Reste $R^1$ bis $R^4$ Wasserstoffatome sind ;
$R^5$ und $R^6$ gleich oder verschieden sind und jeweils Wasserstoffatome, $C_{1-4}$-Alkylreste oder die Gruppe $-(CH_2)_nAr$ darstellen, in der n den Wert 0 bis 4 annimmt und Ar eine gegebenenfalls mit 1 bis 3 Substituenten, ausgewählt aus $C_{1-4}$-Alkyl-, $C_{1-4}$-Alkoxy- oder $C_{1-4}$-Alkylthioreste, Halogenatome, Cyano-, Amino-, Hydroxy-, Carbamoyl- oder Carboxylgruppen, $C_{1-4}$-Alkanoylreste oder Trifluormethylgruppen, substituierte Phenylgruppe bedeutet oder $R^5$ und $R^6$ zusammen mit dem Stickstoffatom, an dem sie gebunden sind, einen gesättigten oder ungesättigten carbocyclischen Ring ergeben ;
und $R^7$ und $R^8$ gleich oder verschieden sind und jeweils Wasserstoffatome, $C_{1-4}$-Alkylreste oder die Gruppe $-(CH_2)_nAr^1$ darstellen, in der n den Wert 0 bis 4 annimmt und $Ar^1$ einen gegebenenfalls mit 1 bis 3 wie vorstehend für Ar beschriebenen Substituenten substituierte Phenylgruppe bedeutet oder $R^7$ und $R^8$ zusammen mit dem Stickstoffatom, an dem sie gebunden sind, einen gesättigten oder ungesättigten carbocyclischen Ring ergeben ;
oder ein pharmazeutisch verträgliches Salz davon, mit der Maßgabe, daß

$R^5$, $R^6$, $R^7$ und $R^8$ nicht alle gleichzeitig Wasserstoffatome sind ;
mindestens einer der Reste $R^5$, $R^6$, $R^7$ und $R^8$ je nach Eignung eine der Gruppen $-(CH_2)_nAr$ oder $-(CH_2)_nAr^1$ bedeutet ; und
falls $R^1$ und $R^4$ beide Wasserstoffatome bedeuten und einer der Reste $R^2$ und $R^3$ ein $C_{1-4}$-Alkoxyrest ist, der andere kein Wasserstoffatom oder $C_{1-4}$-Alkoxyrest ist,
umfassend :

(a) Umsetzung einer Verbindung der Struktur (II)

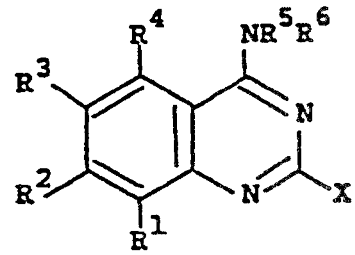

(II)

in der $R^1$ bis $R^6$ wie für Struktur (I) beschrieben sind, ausgenommen daß sie falls erforderlich in geschützter Form vorliegen, und X eine durch ein Ain ersetzbare Gruppe bedeutet, mit einem Amin der Struktur $R^7R^8NH$, in der $R^7$ und $R^8$ wie für Struktur (I) beschrieben sind ; oder

(b) Umsetzung einer Verbindung der Struktur (III)

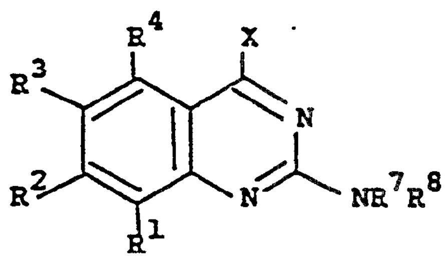

(III)

in der $R^1$ bis $R^4$ und $R^7$ und $R^8$ wie für Struktur (I) beschrieben sind und X eine durch ein Amin ersetzbare Gruppe bedeutet, mit einem Amin der Struktur $R^5R^6NH$, in der $R^5$ und $R^6$ wie für die Struktur (I) beschrieben sind ; und gegebenenfalls hiernach,
Entfernung jeglicher Schutzgruppen ;
Herstellen eines pharmazeutisch verträglichen Salzes.

2. Verfahren nach Anspruch 1(a) falls es in Abwesenheit eines Lösungsmittels in einem verschlossenen Gefäß bei erhöhter Temperatur durchgeführt wird.

3. Verfahren nach Anspruch 1(b) falls es in einem inerten Lösungsmittel bei erhöhter Temperatur durchgeführt wird.

4. Verfahren nach Anspruch 3, wobei das Lösungsmittel ein $C_{1-4}$-Alkanol ist.

5. Verfahren nach einem der Ansprüche 1 bis 4, in dem in der Verbindung der Struktur (I) $R^2$ bis $R^4$ Wasserstoffatome sind und $R^1$ ein Wasserstoffatom oder ein $C_{1-4}$-Alkoxyrest ist.

6. Verfahren nach Anspruch 5, in dem in der Verbindung der Struktur (I) einer der Reste $R^5$ und $R^6$ die Gruppe $-(CH_2)_nAr$ bedeutet, in der n den Wert 0 bis 4 annimmt, und Ar eine gegebenenfalls gemäß Anspruch 1 substituierte Phenylgruppe bedeutet, und der andere ein $C_{1-4}$-Alkylrest ist.

7. Verfahren nach Anspruch 6, in dem in der Verbindung der Struktur (I) n den Wert 0 annimmt.

8. Verfahren nach Anspruch 7, in dem in der Verbindung der Struktur (I) einer der Reste $R^7$ und $R^8$ ein Wasserstoffatom bedeutet und der andere die Gruppe $-(CH_2)_nAr^1$ darstellt, in der n den Wert 0 bis 4 annimmt und $Ar^1$ eine gegebenenfalls gemäß Anspruch 1 substituierte Phenylgruppe bedeutet.

9. Verfahren nach einem der Ansprüche 1 bis 8, in dem die Verbindung der Struktur (I)
2-Amino-8-methoxy-4-(2-methylphenylamino)-chinazolin,
2,4-Bis-(N-methylphenylamino)-chinazolin,
4-(N-Methylphenylamino)-2-(2-methylphenylamino)-8-methoxychinazolin,
2-(2-Methylphenylamino)-4-(N-methylphenylamino)-chinazolin,
2-Phenylamino-4-(N-methylphenylamino)-chinazolin,
2-[(2-Methyl-4-fluorphenyl)-amino]-4-(N-methylphenylamino)-chinazolin oder

2-(2-Methylphenylamino)-4-phenylaminochinazolin, ist.

10. Verfahren zur Herstellung eines Arzneimittels, umfassend das Zusammenbringen einer Verbindung der Struktur (I) oder ein pharmazeutisch verträgliches Salz davon gemäß Anspruch 1 und eines pharmazeutischen Trägers.

11. Verfahren nach Anspruch 10, in dem die Verbindung der Struktur I
2-Amino-8-methoxy-4-(2-methylphenylamino)-chinazolin,
2,4-Bis-(N-methylphenylamino)-chinazolin,
4-(N-Methylphenylamino)-2-(2-methylphenylamino)-8-methoxychinazolin,
2-(2-Methylphenylamino)-4-(N-methylphenylamino)-chinazolin,
2-Phenylamino-4-(N-methylphenylamino)-chinazolin,
2-[(2-Methyl-4-fluorphenyl)-amino]-4-(N-methylphenylamino)-chinazolin oder
2-(2-Methylphenylamino)-4-phenylaminochinazolin,
ist.

12. Verwendung einer Verbindung der Struktur (IA)

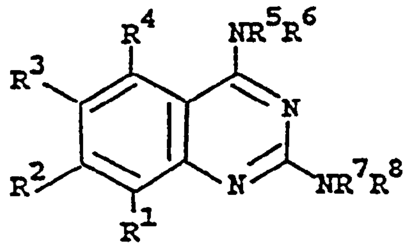

(IA)

in der
$R^1$ bis $R^4$ gleich oder verschieden sind und jeweils Wasserstoffatome, $C_{1-4}$-Alkyl- oder $C_{1-4}$-Alkoxyreste, Phenylgruppen, $C_{1-4}$-Alkylthio- oder $C_{1-4}$-Alkanoylreste, Aminogruppen, $C_{1-6}$-Alkylamino- oder Di-$C_{1-4}$-alkylaminogruppen, Halogenatome oder Trifluormethylgruppen bedeuten, mit der Maßgabe, daß mindestens zwei der Reste $R^1$ bis $R^4$ Wasserstoffatome sind ;
$R^5$ und $R^6$ gleich oder verschieden sind und jeweils Wasserstoffatome, $C_{1-4}$-Alkylreste, oder die Gruppe $-(CH_2)_nAr$ darstellen, in der n die Zahl 0 bis 4 annimmt und Ar eine gegebenenfalls substituierte Phenylgruppe bedeutet oder $R^5$ und $R^6$ zusammen mit dem Stickstoffatom, an dem sie gebunden sind, einen gesättigten oder ungesättigten carbocyclischen Ring ergeben ; und ;
$R^7$ und $R^8$ gleich oder verschieden sind und jeweils Wasserstoffatome, $C_{1-4}$-Alkylreste oder die Gruppe $-(CH_2)_nAr^1$ darstellen, in der n die Zahl von 0 bis 4 annimmt und $Ar^1$ eine gegebenenfalls substituierte Phenylgruppe bedeutet, oder $R^7$ und $R^8$ zusammen mit dem Stickstoffatom, an dem sie gebunden sind, einen gesättigten oder ungesättigten carbocyclischen Ring ergeben ;
oder eines pharmazeutisch verträglichen Salzes davon zur Herstellung eines Arzneimittels zur Behandlung gastrointestinaler Erkrankungen.

**Patentanprüche für den Vertragsstaat : GR**

1. Verfahren zur Herstellung einer Verbindung der Struktur (I)

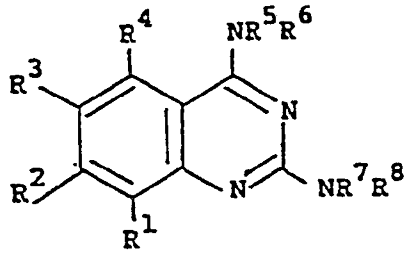

(I)

in der
$R^1$ bis $R^4$ gleich oder verschieden sind und jeweils Wasserstoffatome, $C_{1-4}$-Alkyl- oder $C_{1-4}$-Alkoxyreste, Phenylgruppen, $C_{1-4}$-Alkylthio- oder $C_{1-4}$-Alkanoylreste, Aminogruppen, $C_{1-6}$-Alkylamino- oder Di-$C_{1-4}$-alkylamino-

reste, Halogenatome oder Trifluormethylgruppen bedeuten, mit der Maßgabe, daß mindestens zwei der Reste $R^1$ bis $R^4$ Wasserstoffatome sind ;

$R^5$ und $R^6$ gleich oder verschieden sind und jeweils Wasserstoffatome, $C_{1-4}$-Alkylreste oder die Gruppe $-(CH_2)_nAr$ darstellen, in der n den Wert 0 bis 4 annimmt und Ar eine gegebenenfalls mit 1 bis 3 Substituenten, ausgewählt aus $C_{1-4}$-Alkyl-, $C_{1-4}$-Alkoxy- oder $C_{1-4}$-Alkylthioreste, Halogenatome, Cyano-, Amino-, Hydroxy-, Carbamoyl- oder Carboxylgruppen, $C_{1-4}$-Alkanoylreste oder Trifluormethylgruppen, substituierte Phenylgruppe bedeutet oder $R^5$ und $R^6$ zusammen mit dem Stickstoffatom, an dem sie gebunden sind, einen gesättigten oder ungesättigten carbocyclischen Ring ergeben ;

und $R^7$ und $R^8$ gleich oder verschieden sind und jeweils Wasserstoffatome, $C_{1-4}$-Alkylreste oder die Gruppe $-(CH_2)_nAr^1$ darstellen, in der n den Wert 0 bis 4 annimmt und $Ar^1$ einen gegebenenfalls mit 1 bis 3 wie vorstehend für Ar beschriebenen Substituenten substituierte Phenylgruppe bedeutet oder $R^7$ und $R^8$ zusammen mit dem Stickstoffatom, an dem sie gebunden sind, einen gesättigten oder ungesättigten carbocyclischen Ring ergeben ;

oder ein pharmazeutisch verträgliches Salz davon, mit der Maßgabe, daß

$R^5$, $R^6$, $R^7$ und $R^8$ nicht alle gleichzeitig Wasserstoffatome sind ;

mindestens einer der Reste $R^5$, $R^6$, $R^7$ und $R^8$ je nach Eignung eine der Gruppen $-(CH_2)_nAr$ oder $-(CH_2)_nAr^1$ bedeutet ; und

falls $R^1$ und $R^4$ beide Wasserstoffatome bedeuten und einer der Reste $R^2$ und $R^3$ ein $C_{1-4}$-Alkoxyrest ist, der andere kein Wasserstoffatom oder $C_{1-4}$-Alkoxyrest ist,

umfassend :

(a) Umsetzung einer Verbindung der Struktur (II)

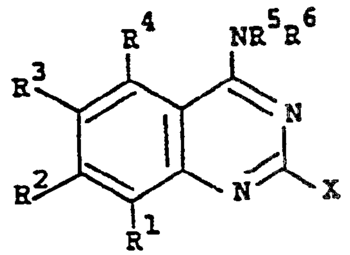

(II)

in der $R^1$ bis $R^6$ wie für Struktur (I) beschrieben sind, ausgenommen daß sie falls erforderlich in geschützter Form vorliegen, und X eine durch ein Amin ersetzbare Gruppe bedeutet, mit einem Amin der Struktur $R^7R^8NH$, in der $R^7$ und $R^8$ wie für Struktur (I) beschrieben sind ; oder

(b) Umsetzung einer Verbindung der Struktur (III)

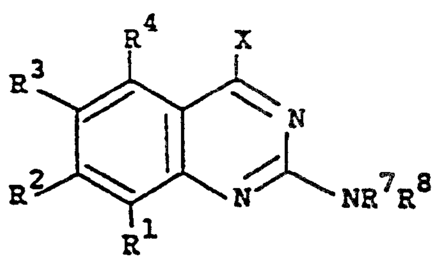

(III)

in der $R^1$ bis $R^4$ und $R^7$ und $R^8$ wie für Struktur (I) beschrieben sind und X eine durch ein Amin ersetzbare Gruppe bedeutet, mit einem Amin der Struktur $R^5R^6NH$, in der $R^5$ und $R^6$ wie für die Struktur (I) beschrieben sind ; und gegebenenfalls hiernach,

Entfernung jeglicher Schutzgruppen ;

Herstellen eines pharmazeutisch verträglichen Salzes.

2. Verfahren nach Anspruch 1(a) falls es in Abwesenheit eines Lösungsmittels in einem verschlossenen Gefäß bei erhöhter Temperatur durchgefürt wird.

3. Verfahren nach Anspruch 1(b) falls es in einem inerten Lösungsmittel bei erhöhter Temperatur durchgeführt wird.

4. Verfahren nach Anspruch 3, wobei das Lösungsmittel ein $C_{1-4}$-Alkanol ist.

5. Verfahren nach einem der Ansprüche 1 bis 4, in dem in der Verbindung der Struktur (I) $R^2$ bis $R^4$ Wasserstoffatome sind und $R^1$ ein Wasserstoffatom oder ein $C_{1-4}$-Alkoxyrest ist.

6. Verfahren nach Anspruch 5, in dem in der Verbindung der Struktur (I) einer der Reste $R^5$ und $R^6$ die

Gruppe $-(CH_2)_nAr$ bedeutet, in der n den Wert 0 bis 4 annimmt, und Ar eine gegebenenfalls gemäß Anspruch 1 substituierte Phenylgruppe bedeutet, und der andere ein $C_{1-4}$-Alkylrest ist.

7. Verfahren nach Anspruch 6, in dem in der Verbindung der Struktur (I) n den Wert 0 annimmt.

8. Verfahren nach Anspruch 7, in dem in der Verbindung der Struktur (I) einer der Reste $R^7$ und $R^8$ ein Wasserstoffatom bedeutet und der andere die Gruppe $-(CH_2)_nAr^1$ darstellt, in der n den Wert 0 bis 4 annimmt und $Ar^1$ eine gegebenenfalls gemäß Anspruch 1 substituierte Phenylgruppe bedeutet.

9. Verfahren nach einem der Ansprüche 1 bis 8, in dem die Verbindung der Struktur I
2-Amino-8-methoxy-4-(2-methylphenylamino)-chinazolin,
2,4-Bis-(N-methylphenylamino)-chinazolin,
4-(N-Methylphenylamino)-2-(2-methylphenylamino)-8-methoxychinazolin,
2-(2-Methylphenylamino)-4-(N-methylphenylamino)-chinazolin,
2-Phenylamino-4-(N-methylphenylamino)-chinazolin,
2-[(2-Methyl-4-fluorphenyl)-amino]-4-(N-methylphenylamino)-chinazolin oder
2-(2-Methylphenylamino)-4-phenylaminochinazolin,
ist.

10. Verfahren zur Herstellung eines Arzneimittels, umfassend das Zusammenbringen einer Verbindung der Struktur (I) oder ein pharmazeutisch verträgliches Salz davon gemäß Anspruch 1 und eines pharmazeutischen Trägers.

11. Verfahren nach Anspruch 10, in dem die Verbindung der Struktur I
2-Amino-8-methoxy-4-(2-methylphenylamino)-chinazolin,
2,4-Bis-(N-methylphenylamino)-chinazolin,
4-(N-Methylphenylamino)-2-(2-methylphenylamino)-8-methoxychinazolin,
2-(2-Methylphenylamino)-4-(N-methylphenylamino)-chinazolin,
2-Phenylamino-4-(N-methylphenylamino)-chinazolin,
2-[(2-Methyl-4-fluorphenyl)-amino]-4-(N-methylphenylamino)-chinazolin oder
2-(2-Methylphenylamino)-4-phenylaminochinazolin,
ist.

12. Verbindung der Struktur (IIA)

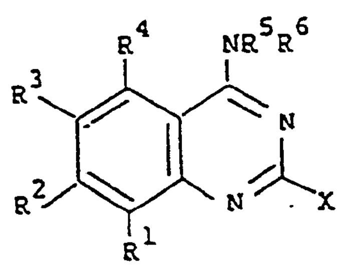

(IIA)

in der $R^1$ bis $R^4$ wie für Struktur (I) gemäß Anspruch 1 beschrieben sind, X eine durch ein Amin ersetzbare Gruppe bedeutet, $R^5$ ein Wasserstoffatom oder einen $C_{1-4}$-Alkylrest bedeutet und $R^6$ die Gruppe $(CH_2)_nAr$ darstellt, in der n und Ar wie für Struktur (I) beschrieben sind.

13. Verbindung der Struktur (IIIA)

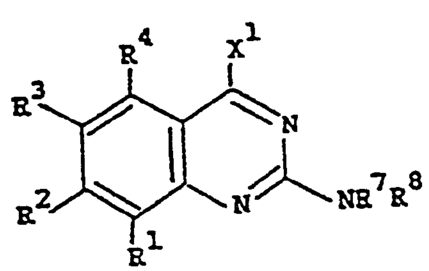

(IIIA)

in der $R^1$ bis $R^4$ wie für Struktur (I) gemäß Anspruch 1 beschrieben sind, X eine durch ein Amin ersetzbare Gruppe bedeutet, $R^7$ ein Wasserstoffatom oder einen $C_{1-4}$-Alkylrest bedeutet und $R^8$ die Gruppe $(CH_2)_nAr^1$ darstellt, in der n und $Ar^1$ wie für Struktur (I) beschrieben sind.

14. Verwendung einer Verbindung der Struktur (IA)

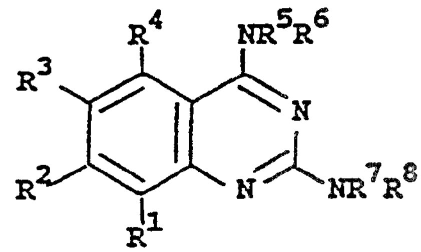

(IA)

in der
$R^1$ bis $R^4$ gleich oder verschieden sind und jeweils Wasserstoffatome, $C_{1-4}$-Alkyl- oder $C_{1-4}$-Alkoxyreste, Phenylgruppen, $C_{1-4}$-Alkylthio- oder $C_{1-4}$-Alkanoylreste, Aminogruppen, $C_{1-6}$-Alkylamino- oder Di-$C_{1-4}$-alkylaminogruppen, Halogenatome oder Trifluormethylgruppen bedeuten, mit der Maßgabe, daß mindestens zwei der Reste $R^1$ bis $R^4$ Wasserstoffatome sind ;
$R^5$ und $R^6$ gleich oder verschieden sind und jeweils Wasserstoffatome, $C_{1-4}$-Alkylreste, oder die Gruppe $-(CH_2)_nAr$ darstellen, in der n die Zahl 0 bis 4 annimmt und Ar eine gegebenenfalls substituierte Phenylgruppe bedeutet oder $R^5$ und $R^6$ zusammen mit dem Stickstoffatom, an dem sie gebunden sind, einen gesättigten oder ungesättigten carbocyclischen Ring ergeben ; und ;
$R^7$ und $R^8$ gleich oder verschieden sind und jeweils Wasserstoffatome, $C_{1-4}$-Alkylreste oder die Gruppe $-(CH_2)_nAr^1$ darstellen, in der n die Zahl von 0 bis 4 annimmt und $Ar^1$ eine gegebenenfalls substituierte Phenylgruppe bedeutet, oder $R^7$ und $R^8$ zusammen mit dem Stickstoffatom, an dem sie gebunden sind, einen gesättigten oder ungesättigten carbocyclischen Ring ergeben ;
oder eines pharmazeutisch verträglichen Salzes davon zur Herstellung eines Arzneimittels zur Behandlung gastrointestinaler Erkrankungen.

**Revendications**

**Revendications pour les Etats Contractants : AT, BE, CH, DE, FR, GB, IT, LI, LU, NL, SE**

1. Composé de structure (I)

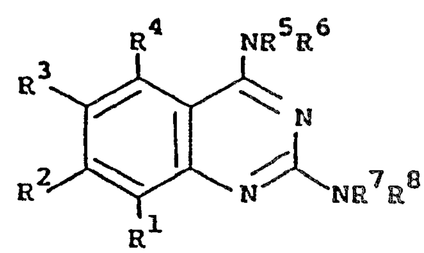

(I)

dans laquelle
$R^1$ à $R^4$ sont semblables ou différents et représentent chacun un atome d'hydrogène ou un groupe alkyle en $C_{1-4}$, alcoxy en $C_{1-4}$, phényle, alkylthio en $C_{1-4}$, alcanoyle en $C_{1-4}$, amino, alkylamino en $C_{1-6}$, di(alkyl en $C_{1-4}$)amino, halogéno ou trifluorométhyle, sous réserve qu'au moins deux de $R^1$ à $R^4$ représentent un atome d'hydrogène ;
$R^5$ et $R^6$ sont semblables ou différents et représentent chacun un atome d'hydrogène ou un groupe alkyle en $C_{1-4}$ ou $-(CH_2)_nAr$, dans lequel n a une valeur de 0 à 4 et Ar représente un groupe phényle facultativement substitué par 1 à 3 substituants choisis parmi les groupes alkyle en $C_{1-4}$, alcoxy en $C_{1-4}$, alkylthio en $C_{1-4}$, halogéno, cyano, amino, hydroxy, carbamoyle, carboxy, alcanoyle en $C_{1-4}$ ou trifluorométhyle, ou $R^5$ et $R^6$, avec l'atome d'azote auquel ils sont fixés, forment un cycle carbocyclique saturé ou insaturé ;
et
$R^7$ et $R^8$ sont semblables ou différents et représentent chacun un atome d'hydrogène ou un groupe alkyle en $C_{1-4}$ ou $-(CH_2)_nAr^1$, dans lequel n a une valeur de 0 à 4 et $Ar^1$ représente un groupe phényle facultativement substitué par 1 à 3 substituants, comme décrit pour Ar ci-dessus, ou $R^7$ et $R^8$, avec l'atome d'azote auquel ils sont fixés, forment un cycle carbocyclique saturé ou insaturé ; ou un de ses sels pharmaceutiquement acceptables, sous réserve que :
° $R^5$, $R^6$, $R^7$ et $R^8$ ne représentent pas tous simultanément un atome d'hydrogène ;

° au moins un de $R^5$, $R^6$, $R^7$ et $R^8$ représente un groupe $-(CH_2)_nAr$ ou $-(CH_2)_nAr^1$ selon le cas ; et

° lorsque $R^1$ et $R^4$ représentent chacun un atome d'hydrogène et un de $R^2$ et $R^3$ représente un groupe alcoxy en $C_{1-4}$, l'autre ne représente ni un atome d'hydrogène ni un groupe alcoxy en $C_{1-4}$.

2. Composé selon la revendication 1, dans lequel $R^2$ à $R^4$ représentent un atome d'hydrogène et $R^1$ représente un atome d'hydrogène ou un groupe alcoxy en $C_{1-4}$.

3. Composé selon la revendication 2, dans lequel un de $R^5$ et $R^6$ représente $-(CH_2)_nAr$, où n a une valeur de 0 à 4 et Ar représente un groupe phényle, facultativement substitué comme défini dans la revendication 1, et l'autre représente un groupe alkyle en $C_{1-4}$.

4. Composé selon la revendication 3, dans lequel n est 0.

5. Composé selon la revendication 4, dans lequel un de $R^7$ et $R^8$ représente un atome d'hydrogène et l'autre représente $-(CH_2)_nAr^1$, où n a une valeur de 0 à 4 et $Ar^1$ représente un cycle phényle facultativement substitué comme défini dans la revendication 1.

6. Composé selon la revendication 1, qui est
la 2-amino-8-méthoxy-4-(2-méthylphénylamino)quinazoline,
la 2,4-bis(N-méthylphénylamino)quinazoline,
la 4-(N-méthylphénylamino)-2-(2-méthylphénylamino)-8-méthoxyquinazoline,
la 2-(2-méthylphénylamino)-4-(N-méthylphénylamino)quinazoline,
la 2-phénylamino-4-(N-méthylphénylamino)quinazoline,
la 2-[(2-méthyl-4-fluorophényl)amino]-4-(N-méthylphénylamino)quinazoline,
la 2-(2-méthylphénylamino)-4-phénylaminoquinazoline,
ou un sel pharmaceutiquement acceptable de celles-ci.

7. Composition pharmaceutique comprenant un composé selon l'une quelconque des revendications 1 à 6 et un véhicule pharmaceutique.

8. Composé selon l'une quelconque des revendications 1 à 6 pour l'emploi comme agent thérapeutique.

9. Procédé pour la préparation d'un composé selon la revendication 1, qui comprend :

(a) la réaction d'un composé de structure (II)

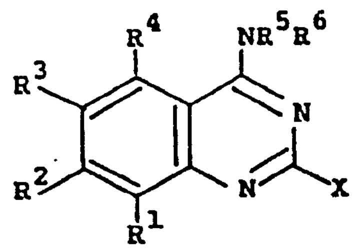

(II)

dans laquelle $R^1$ à $R^6$ sont comme décrit pour la structure (I), si ce n'est que, le cas échéant, ils sont sous une forme protégée, et X représente un groupe déplaçable par une amine, avec une amine de structure $R^7R^8NH$, dans laquelle $R^7$ et $R^8$ sont comme décrit pour la structure (I) ; ou

(b) la réaction d'un composé de structure (III)

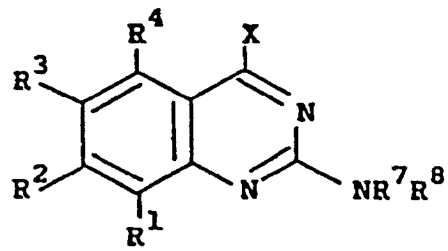

(III)

dans laquelle $R^1$ à $R^4$ et $R^7$ et $R^8$ sont comme décrit pour la structure (I) et X représente un groupe déplaçable par une amine, avec une amine de structure $R^5R^6NH$, dans laquelle $R^5$ et $R^6$ sont comme décrit pour la structure (I) ; puis, facultativement,

° l'élimination de tout groupe protecteur ; et

° la formation d'un sel pharmaceutiquement acceptable.

10. Composé de structure (II)

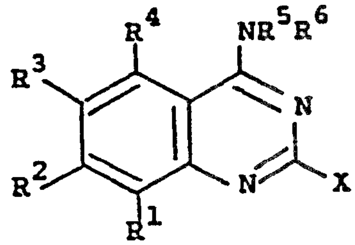

(II)

dans laquelle $R^1$ à $R^6$ sont comme décrit pour la structure (I) dans la revendication 1 et X représente un groupe déplaçable par une amine.

11. Composé de structure (III)

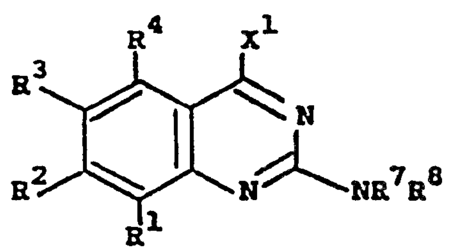

(III)

dans laquelle $R^1$ à $R^4$ et $R^7$ et $R^8$ sont comme décrit pour la structure (I) dans la revendication 1 et X représente un groupe déplaçable par une amine.

12. Utilisation d'un composé de structure (IA)

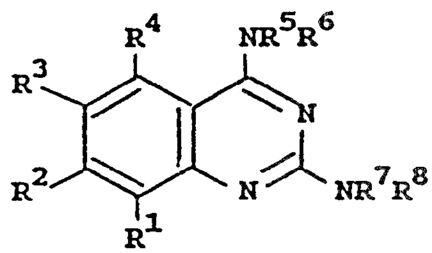

(IA)

dans laquelle

$R^1$ à $R^4$ sont semblables ou différents et représentent chacun un atome d'hydrogène ou un groupe alkyle en $C_{1-4}$, alcoxy en $C_{1-4}$, phényle, alkylthio en $C_{1-4}$, alcanoyle en $C_{1-4}$, amino, alkylamino en $C_{1-6}$, di(alkyl en $C_{1-4}$)amino, halogéno ou trifluorométhyle, sous réserve qu'au moins deux de $R^1$ à $R^4$ représentent un atome d'hydrogène ;

$R^5$ et $R^6$ sont semblables ou différents et représentent chacun un atome d'hydrogène ou un groupe alkyle en $C_{1-4}$ ou $-(CH_2)_nAr$, dans lequel n a une valeur de 0 à 4 et Ar représente un groupe phényle facultativement substitué, ou $R^5$ et $R^6$, avec l'atome d'azote auquel ils sont fixés, forment un cycle carbocyclique saturé ou insaturé ;

et

$R^7$ et $R^8$ sont semblables ou différents et représentent chacun un atome d'hydrogène ou un groupe alkyle en $C_{1-4}$ ou $-(CH_2)_nAr^1$, dans lequel n a une valeur de 0 à 4 et $Ar^1$ représente un groupe phényle facultativement substitué, ou $R^7$ et $R^8$, avec l'atome d'azote auquel ils sont fixés, forment un cycle carbocyclique saturé ou insaturé ;

ou un sel pharmaceutiquement acceptable de celui-ci pour la fabrication d'un médicament pour le traitement des maladies gastrointestinales.

**Revendications pour l'Etat Contractant : ES.**

1. Procédé pour la préparation d'un composé de structure (I)

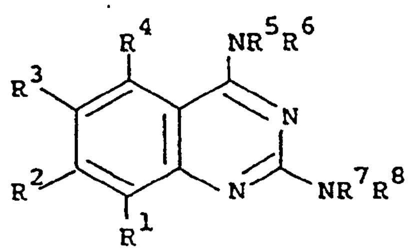

(I)

dans laquelle
$R^1$ à $R^4$ sont semblables ou différents et représentent chacun un atome d'hydrogène ou un groupe alkyle en $C_{1-4}$, alcoxy en $C_{1-4}$, phényle, alkylthio en $C_{1-4}$, alcanoyle en $C_{1-4}$, amino, alkylamino en $C_{1-6}$, di(alkyl en $C_{1-4}$)amino, halogéno ou trifluorométhyle, sous réserve qu'au moins deux de $R^1$ à $R^4$ représentent un atome d'hydrogène ;
$R^5$ et $R^6$ sont semblables ou différents et représentent chacun un atome d'hydrogène ou un groupe alkyle en $C_{1-4}$ ou $-(CH_2)_nAr$, dans lequel n a une valeur de 0 à 4 et Ar représente un groupe phényle facultativement substitué par 1 à 3 substituants choisis parmi les groupes alkyle en $C_{1-4}$, alcoxy en $C_{1-4}$, alkylthio en $C_{1-4}$, halogéno, cyano, amino, hydroxy, carbamoyle, carboxy, alcanoyle en $C_{1-4}$ ou trifluorométhyle, ou $R^5$ et $R^6$, avec l'atome d'azote auquel ils sont fixés, forment un cycle carbocyclique saturé ou insaturé ;
et
$R^7$ et $R^8$ sont semblables ou différents et représentent chacun un atome d'hydrogène ou un groupe alkyle en $C_{1-4}$ ou $-(CH_2)_nAr^1$, dans lequel n a une valeur de 0 à 4 et $Ar^1$ représente un groupe phényle facultativement substitué par 1 à 3 substituants, comme décrit pour Ar ci-dessus, ou $R^7$ et $R^8$, avec l'atome d'azote auquel ils sont fixés, forment un cycle carbocyclique saturé ou insaturé ; ou un de ses sels pharmaceutiquement acceptables, sous réserve que :
° $R^5$, $R^6$, $R^7$ et $R^8$ ne représentent pas tous simultanément un atome d'hydrogène ;
° au moins un de $R^5$, $R^6$, $R^7$ et $R^8$ représente un groupe $-(CH_2)_nAr$ ou $-(CH_2)_nAr^1$ selon le cas ; et
° lorsque $R^1$ et $R^4$ représentent chacun un atome d'hydrogène et un de $R^2$ et $R^3$ représente un groupe alcoxy en $C_{1-4}$, l'autre ne représente ni un atome d'hydrogène ni un groupe alcoxy en $C_{1-4}$,
qui comprend
(a) la réaction d'un composé de structure (II)

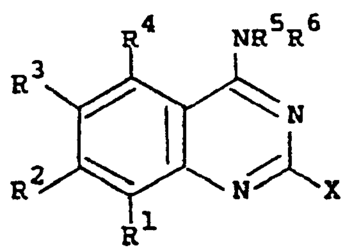

(II)

dans laquelle $R^1$ à $R^6$ sont comme décrit pour la structure (I), si ce n'est que, le cas échéant, ils sont sous une forme protégée, et X représente un groupe déplaçable par une amine, avec une amine de structure $R^7R^8NH$, dans laquelle $R^7$ et $R^8$ sont comme décrit pour la structure (I) ; ou
(b) la réaction d'un composé de structure (III)

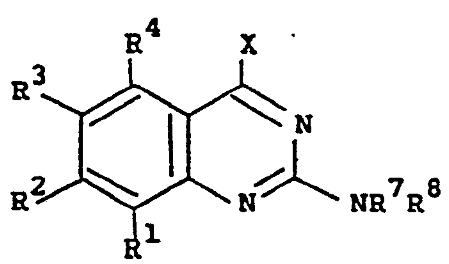

(III)

dans laquelle $R^1$ à $R^4$ et $R^7$ et $R^8$ sont comme décrit pour la structure (I) et X représente un groupe déplaçable par une amine, avec une amine de structure $R^5R^6NH$, dans laquelle $R^5$ et $R^6$ sont comme décrit pour

la structure (I) ; puis, facultativement,

° l'élimination de tout groupe protecteur ; et

° la formation d'un sel pharmaceutiquement acceptable

2. procédé selon la revendication 1(a) effectué en l'absence de solvant dans un récipient fermé à température élevée.

3. Procédé selon la revendication 1(b) effectué dans un solvant inerte à température élevée.

4. Procédé selon la revendication 3, dans lequel le solvant est un alcanol en $C_{1-4}$.

5. Procédé selon l'une quelconque des revendications 1 à 4, dans lequel, dans le composé de structure (I), $R^2$ à $R^4$ représentent un atome d'hydrogène et $R^1$ représente un atome d'hydrogène ou un groupe alcoxy en $C_{1-4}$.

6. Procédé selon la revendication 5, dans lequel, dans le composé de structure (I), un de $R^5$ et $R^6$ représente $-(CH_2)_nAr$, où n a une valeur de 0 à 4 et Ar représente un cycle phényle facultativement substitué, comme défini dans la revendication 1, et l'autre représente un groupe alkyle en $C_{1-4}$.

7. Procédé selon la revendication 6, dans lequel, dans le composé de structure (I), n est 0.

8. Procédé selon la revendication 7, dans lequel, dans le composé de structure (I), un de $R^7$ et $R^8$ représente un atome d'hydrogène et l'autre représente $-(CH_2)_nAr^1$, où n a une valeur de 0 à 4 et $Ar^1$ représente un cycle phényle facultativement substitué, comme défini dans la revendication 1.

9. Procédé selon l'une quelconque des revendication 1 à 8, dans lequel le composé de structure (I) est
la 2-amino-8-méthoxy-4-(2-méthylphénylamino)quinazoline,
la 2,4-bis(N-méthylphénylamino)quinazoline,
la 4-(N-méthylphénylamino)-2-(2-méthylphénylamino)-8-méthoxyquinazoline,
la 2-(2-méthylphénylamino)-4-(N-méthylphénylamino)quinazoline,
la 2-phénylamino-4-(N-méthylphénylamino)quinazoline,
la 2-[(2-méthyl-4-fluorophényl)amino]-4-(N-méthylphénylamino)quinazoline, et
la 2-(2-méthylphénylamino)-4-phénylaminoquinazoline.

10. Procédé pour la préparation d'une composition pharmaceutique, qui comprend l'association d'un composé de structure (I) ou d'un sel pharmaceutiquement acceptable de celui-ci, comme décrit dans la revendication 1, et d'un véhicule pharmaceutique.

11. Procédé selon la revendication 10, dans lequel le composé de structure (I) est
la 2-amino-8-méthoxy-4-(2-méthylphénylamino)quinazoline,
la 2,4-bis(N-méthylphénylamino)quinazoline,
la 4-(N-méthylphénylamino)-2-(2-méthylphénylamino)-8-méthoxyquinazoline,
la 2-(2-méthylphénylamino)-4-(N-méthylphénylamino)quinazoline,
la 2-phénylamino-4-(N-méthylphénylamino)quinazoline,
la 2-[(2-méthyl-4-fluorophényl)amino]-4-(N-méthylphénylamino)quinazoline, et
la 2-(2-méthylphénylamino)-4-phénylaminoquinazoline.

12. Utilisation d'un composé de structure (IA)

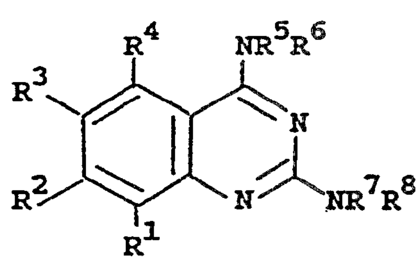

(IA)

dans laquelle

$R^1$ à $R^4$ sont semblables ou différents et représentent chacun un atome d'hydrogène ou un groupe alkyle en $C_{1-4}$, alcoxy en $C_{1-4}$, phényle, alkylthio en $C_{1-4}$, alcanoyle en $C_{1-4}$, amino, alkylamino en $C_{1-6}$, di(alkyl en $C_{1-4}$)amino, halogéno ou trifluorométhyle, sous réserve qu'au moins deux de $R^1$1 à $R^4$ représentent un atome d'hydrogène ;

$R^5$ et $R^6$ sont semblables ou différents et représentent chacun un atome d'hydrogène ou un groupe alkyle en $C_{1-4}$ ou $-(CH_2)_nAr^1$, dans lequel n a une valeur de 0 à 4 et $Ar^1$ représente un groupe phényle facultativement substitué, ou $R^5$ et $R^6$, avec l'atome d'azote auquel ils sont fixés, forment un cycle carbocyclique saturé ou insaturé ;

et

$R^7$ et $R^8$ sont semblables ou différents et représentent chacun un atome d'hydrogène ou un groupe alkyle en

$C_{1-4}$ ou $-(CH_2)_nAr^1$, dans lequel n a une valeur de 0 à 4 et $Ar^1$ représente un groupe phényle facultativement substitué, ou $R^7$ et $R^8$, avec l'atome d'azote auquel ils sont fixés, forment un cycle carbocyclique saturé ou insaturé ; ou un de ses sels pharmaceutiquement acceptables, pour la fabrication d'un médicament pour le traitement des maladies gastro-intestinales.

**Revendications pour l'Etat Contractant : GR.**

1. Procédé pour la préparation d'un composé de structure (I)

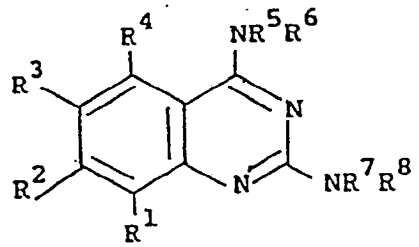

(I)

dans laquelle
$R^1$ à $R^4$ sont semblables ou différents et représentent chacun un atome d'hydrogène ou un groupe alkyle en $C_{1-4}$, alcoxy en $C_{1-4}$, phényle, alkylthio en $C_{1-4}$, alcanoyle en $C_{1-4}$, amino, alkylaino en $C_{1-6}$, di(alkyl en $C_{1-4}$)amino, halogéno ou trifluorométhyle, sous réserve qu'au moins deux de $R^1$ à $R^4$ représentent un atome d'hydrogène ;
$R^5$ et $R^6$ sont semblables ou différents et représentent chacun un atome d'hydrogène ou un groupe alkyle en $C_{1-4}$ ou $-(CH_2)_nAr$, dans lequel n a une valeur de 0 à 4 et Ar représente un groupe phényle facultativement substitué par 1 à 3 substituants choisis parmi les groupes alkyle en $C_{1-4}$, alcoxy en $C_{1-4}$, alkylthio en $C_{1-4}$, halogéno, cyano, amino, hydroxy, carbamoyle, carboxy, alcanoyle en $C_{1-4}$ ou trifluorométhyle, ou $R^5$ et $R^6$, avec l'atome d'azote auquel ils sont fixés, forment un cycle carbocyclique saturé ou insaturé ;
et
$R^7$ et $R^8$ sont semblables ou différents et représentent chacun un atome d'hydrogène ou un groupe alkyle en $C_{1-4}$ ou $-(CH_2)_nAr^1$, dans lequel n a une valeur de 0 à 4 et $Ar^1$ représente un groupe phényle facultativement substitué par 1 à 3 substituants, comme décrit pour Ar ci-dessus, ou $R^7$ et $R^8$, avec l'atome d'azote auquel ils sont fixés, forment un cycle carbocyclique saturé ou insaturé ; ou un de ses sels pharmaceutiquement acceptables, sous réserve que :
° $R^5$, $R^6$, $R^7$ et $R^8$ ne représentent pas tous simultanément un atome d'hydrogène ;
° au moins un de $R^5$, $R^6$, $R^7$ et $R^8$ représente un groupe $-(CH_2)_nAr$ ou $-(CH_2)_nAr^1$ selon le cas ; et
° lorsque $R^1$ et $R^4$ représentent chacun un atome d'hydrogène et un de $R^2$ et $R^3$ représente un groupe alcoxy en $C_{1-4}$, l'autre ne représente ni un atome d'hydrogène ni un groupe alcoxy en $C_{1-4}$,
qui comprend

(a) la réaction d'un composé de structure (II)

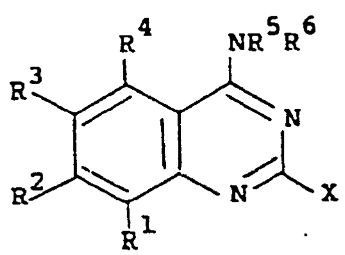

(II)

dans laquelle $R^1$ à $R^6$ sont comme décrit pour la structure (I), si ce n'est que, le cas échéant, ils sont sous une forme protégée, et X représente un groupe déplaçable par une amine, avec une amine de structure $R^7R^8NH$, dans laquelle $R^7$ et $R^8$ sont comme décrit pour la structure (I) ; ou

(b) la réaction d'un composé de structure (III)

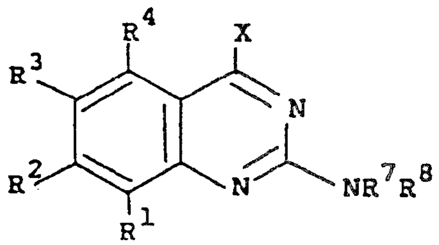

(III)

dans laquelle $R^1$ à $R^4$ et $R^7$ et $R^8$ sont comme décrit pour la structure (I) et X représente un groupe déplaçable par une amine, avec une amine de structure $R^5R^6NH$, dans laquelle $R^5$ et $R^6$ sont comme décrit pour la structure (I) ; puis, facultativement,

° l'élimination de tout groupe protecteur ; et

° la formation d'un sel pharmaceutiquement acceptable

2. Procédé selon la revendication 1(a) effectué en l'absence de solvant dans un récipient fermé à température élevée.

3. Procédé selon la revendication 1(b) effectué dans un solvant inerte à température élevée.

4. Procédé selon la revendication 3, dans lequel le solvant est un alcanol en $C_{1-4}$.

5. Procédé selon l'une quelconque des revendications 1 à 4, dans lequel, dans le composé de structure (I), $R^2$ à $R^4$ représentent un atome d'hydrogène et $R^1$ représente un atome d'hydrogène ou un groupe alcoxy en $C_{1-4}$.

6. Procédé selon la revendication 5, dans lequel, dans le composé de structure (I), un de $R^5$ et $R^6$ représente $-(CH_2)_n,Ar^1$, où n a une valeur de 0 à 4 et Ar représente un cycle phényle facultativement substitué, comme défini dans la revendication 1, et l'autre représente un groupe alkyle en $C_{1-4}$.

7. Procédé selon la revendication 6, dans lequel, dans le composé de structure (I), n est 0.

8. Procédé selon la revendication 7, dans lequel, dans le composé de structure (I), un de $R^7$ et $R^8$ représente un atome d'hydrogène et l'autre représente $-(CH_2)_nAr^1$, où n a une valeur de 0 à 4 et $Ar^1$ représentent un cycle phényle facultativement substitué, comme défini dans la revendication 1.

9. Procédé selon l'une quelconque des revendication 1 à 8, dans lequel le composé de structure (I) est
la 2-amino-8-méthoxy-4-(2-méthylphénylamino)quinazoline, la 2,4-bis(N-méthylphénylamino)quinazoline,
la 4-(N-méthylphénylamino)-2-(2-méthylphénylamino)-8-méthoxyquinazoline,
la 2-(2-méthylphénylamino)-4-(N-méthylphénylamino)quinazoline,
la 2-phénylamino-4-(N-méthylphénylamino)quinazoline,
la 2-[(2-méthyl-4-fluorophényl)amino]-4-(N-méthylphénylamino)quinazoline, et
la 2-(2-méthylphénylamino)-4-phénylaminoquinazoline.

10. Procédé pour la préparation d'une composition pharmaceutique, qui comprend l'association d'un composé de structure (I) ou d'un sel pharmaceutiquement acceptable de celui-ci, comme décrit dans la revendication 1, et d'un véhicule pharmaceutique.

11. Procédé selon la revendication 10, dans lequel le composé de structure (I) est
la 2-amino-8-méthoxy-4-(2-méthylphénylamino)quinazoline,
la 2,4-bis(N-méthylphénylamino)quinazoline,
la 4-(N-méthylphénylamino)-2-(2-méthylphénylamino)-8-méthoxyquinazoline,
la 2-(2-méthylphénylamino)-4-(N-méthylphénylamino)quinazoline,
la 2-phénylamino-4-(N-méthylphénylamino)quinazoline,
la 2-[(2-méthyl-4-fluorophényl)amino]--4-(N-méthylphénylamino)quinazoline, et
la 2-(2-méthylphénylamino)-4-phénylaminoquinazoline.

12. Composé de structure (IIA)

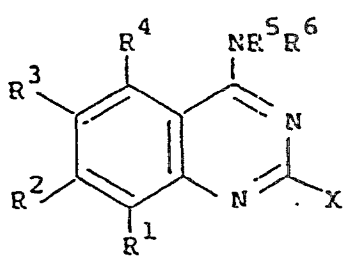

(IIA)

dans laquelle $R^1$ à $R^4$ sont comme décrit pour la structure (I) dans la revendication 1, X représente un groupe déplaçable par une amine, $R^5$ représente un atome d'hydrogène ou un groupe alkyle en $C_{1-4}$ et $R^6$ représente $-(CH_2)_nAr$, où n et Ar sont comme décrit pour la structure (I).

13. Composé de structure (IIIA)

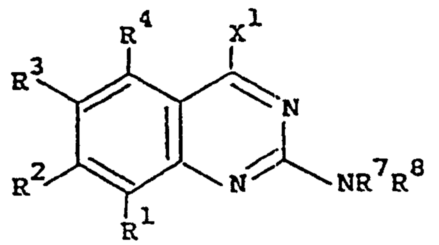

(IIIA)

dans laquelle $R^1$ à $R^4$ sont comme décrit pour la structure (I) dans la revendication 1, X représente un groupe déplaçable par une amine, $R^7$ représente un atome d'hydrogène ou un groupe alkyle en $C_{1-4}$ et $R^8$ représente $-(CH_2)_nAr^1$, où n et $Ar^1$ sont comme décrit pour la structure (I).

14. Utilisation d'un composé de structure (IA)

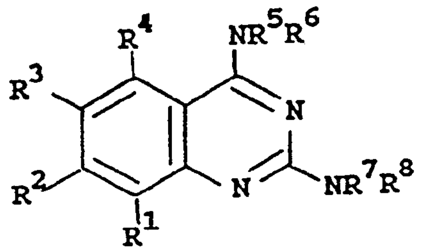

(IA)

dans laquelle

$R^1$ à $R^4$ sont semblables ou différents et représentent chacun un atome d'hydrogène ou un groupe alkyle en $C_{1-4}$, alcoxy en $C_{1-4}$, phényle, alkylthio en $C_{1-4}$, alcanoyle en $C_{1-4}$, amino, alkylamino en $C_{1-6}$, di(alkyl en $C_{1-4}$)amino, halogéno ou trifluorométhyle, sous réserve qu'au moins deux de $R^1$ à $R^4$ représentent un atome d'hydrogène ;

$R^5$ et $R^6$ sont semblables ou différents et représentent chacun un atome d'hydrogène ou un groupe alkyle en $C_{1-4}$ ou $-(CH_2)_nAr$, dans lequel n a une valeur de 0 à 4 et Ar représente un groupe phényle facultativement substitué, ou $R^5$ et $R^6$, avec l'atome d'azote auquel ils sont fixés, forment un cycle carbocyclique saturé ou insaturé ;

et

$R^7$ et $R^8$ sont semblables ou différents et représentent chacun un atome d'hydrogène ou un groupe alkyle en $C_{1-4}$ ou $-(CH_2)_nAr^1$, dans lequel n a une valeur de 0 à 4 et $Ar^1$ représente un groupe phényle facultativement substitué, ou $R^7$ et $R^8$, avec l'atome d'azote auquel ils sont fixés, forment un cycle carbocyclique saturé ou insaturé ; ou un de ses sels pharmaceutiquement acceptables pour la fabrication d'un médicament pour le traitement des maladies gastro-intestinales.